# EUROPEAN PATENT APPLICATION

(11) **EP 4 338 787 A1**
(43) Date of publication of application: **20.03.2024**
(21) Application number: 23196659.9
(22) Date of filing: 11.09.2023
(51) Int. Cl.: A61M 60/139, A61M 60/295, A61M 60/843, A61M 60/865

(54) **A BLOOD PUMP SUPPORT APPARATUS AND METHOD FOR A BLOOD PUMP ASSEMBLY**

(30) Priority: 13.09.2022 US 202217944125
(71) Applicant: NuPulseCV, Inc., Raleigh, NC 27607-4163 (US)
(72) Inventor: Hall, Robert Christopher, Raleigh (US); Woolley, Joshua Ryan, Raleigh (US); Giridharan, Guruprasad Anapathur, Raleigh (US); Pinto, Duane Sidney, Raleigh (US)
(74) Representative: Forresters IP LLP

(57) **Abstract**

A blood pump assembly has a balloon disposed at a distal region and a driveline disposed at a proximal region and is capable of being employed to provide mechanical circulatory support (e.g., counterpulsation). A blood pump support apparatus has a support structure with a head region and a tail region that is removably disposable in the blood pump assembly. When the support structure is disposed in the blood pump assembly, the head region is disposed within the balloon. When the blood pump assembly is implanted in the descending aorta and the support structure is disposed in the blood pump assembly, forces act on the balloon that would cause the balloon to roll or fold upon itself along an angle with respect to the balloon's longitudinal axis. The head region of support structure opposes these forces.

## Description

### TECHNICAL FIELD

The present technology is directed to mechanical circulatory support devices and, in particular, to blood pump assemblies and associated devices, systems and methods.

### BACKGROUND

The prevalence of heart failure (HF) is increasing worldwide and is an expensive burden on health care providers. Despite advances in medical care, prognosis with HF remains poor, especially in advanced stages. Heart transplantation remains limited by the supply of donor organs. The use of left ventricular assist devices (LVAD) is stagnant at approximately 5,000 implants per year due to, among other things, the need for major operative intervention and the use of cardiopulmonary bypass (CPB). Additionally, the high cost of these devices has prevented adoption in large potential markets, with some countries deciding not to fund the use of chronic LVADs.

Globally, the most common assist device for acute heart failure is the intra-aortic balloon pump (IABP), which is used clinically for limited time periods of several hours to several days. An IABP (also referred to herein as a "blood pump," a "balloon" or an "expandable member") is part of an IABP assembly which includes a driveline with two ends. One end is coupleable to the IABP and the other end is, often indirectly, coupleable to an external drive unit. The drive unit is the source of the working fluid (e.g., ambient air or helium), which is carried via the driveline to the IABP for inflation. The drive unit is also responsible for the deflation of the working fluid from the IABP, again via the driveline. Each year more than 150,000 patients worldwide receive IABP therapy. IABPs are much simpler than current LVADs, and the therapeutic effectiveness of counterpulsation therapy is well established. Counterpulsation requires no direct cannulation of the heart leading to easier implantation and explantation. Counterpulsation therapy is also less expensive compared to LVAD therapy.

Counterpulsation therapy is achieved by rapidly inflating the balloon immediately after aortic valve closure (dicrotic notch) and rapidly deflating the balloon just before the onset of systole. The dicrotic notch may be detected using a pressure sensor disposed at the tip of the IABP and the onset of systole may be detected using an electrocardiogram (ECG). Inflation and deflation, however, may both be triggered by either pressure sensor or ECG data. The rapid inflation of the balloon increases the diastolic aortic pressure by 20-70%, improving end-organ and coronary perfusion. The rapid deflation of the balloon reduces the ejection pressure of the native ventricle, reducing afterload and left ventricular external work. Counterpulsation therapy has been shown to be most effective in patients when their systolic aortic pressures are between 40-70 mmHg, native heart rates between 80-110 bpm, and when the counterpulsation volume (i.e., balloon volume) equals the stroke volume of the native left ventricle.

IABPs have been used in HF patients awaiting transplant and in patients undergoing coronary artery bypass surgery. The balloon is generally implanted in the descending aorta with the driveline extending through the femoral artery. This implantation being sometimes referred to herein as the "femoral implantation" and the process resulting in the femoral implantation being sometimes referred to herein as the "femoral technique." The femoral technique is a simple one as there are no significant arterial tortuosity or arterial curvature for the implanting clinician to deal with. However, IABPs implanted via the femoral technique require the patient to remain supine for the duration of therapy with the leg immobilized because (1) changes in orientation have been shown to diminish the effectiveness of therapy, (2) ambulation may cause the balloon or balloon driveline to kink due to movement of the leg leading to cyclic fatigue and ultimately failure of the balloon assembly, and (3) ambulation increases the risk of bleeding at the arterial access in the femoral artery. Consequently, patients cannot walk, or benefit from the IABP as an extended therapy for myocardial support. The lack of ambulation has been demonstrated to lead to poorer outcomes and prolong patient recovery and hospitalization. In addition to arterial access, biocompatibility, and durability issues limit the application of IABP to short durations (typically 2-4 days). While IABP support has been used for prolonged periods, the frequency of vascular complications, infections and bleeding are high.

More recently, IABPs have been implanted in the descending aorta with the driveline extending through the axillary or subclavian artery. This implantation being sometimes referred to herein as the "axillary implantation" and the process resulting in the axillary implantation being sometimes referred to herein as the "axillary technique." When the IABP is implanted using the axillary technique, certain mobility issues related to femoral implantation are mitigated, facilitating patient ambulation. However, with axillary implantation, the natural buoyancy of the balloon when inflated can cause the balloon to flex and the balloon may undergo cyclic fatigue and fail. Axillary implantation has typically been performed surgically via entering the chest and cutting down to the axillary/subclavian artery.

Applicant/Assignee NuPulseCV, Inc. of Raleigh, North Carolina has developed a percutaneously-delivered intravascular ventricular assist system (PiVAS) that functions as a chronic counterpulsation device as generally described in U.S. Patent Application Serial No. 16/876,110, the contents of which are incorporated herein by reference in its entirety. The PiVAS includes an expandable member that is implanted in the descending aorta via an improved axillary technique using minimally invasive surgical techniques . When implanted, the driveline of the PiVAS extends through the axillary or subclavian artery. The PiVAS offers an alternative therapy for HF patients by providing partial circulatory support that may be implanted minimally invasively without entering the chest and does not require cardiopulmonary bypass (CPB) or blood products.

In one embodiment, the PiVAS includes a pneumatically driven expandable member that is designed for long-term biocompatibility and safety. The proximal end of the expandable member may be coupled to a distal end of a driveline. In one embodiment, the proximal end of the expandable member may include an engagement region that is sized and shaped to fit over a distal end of the driveline. An attachment feature such as a compression ring or other suitable element providing an airtight connection/pneumatic seal between the engagement region and driveline may be used to couple the expandable member to the driveline. The proximal end of a driveline may be coupled to a drive unit (e.g., an external driver).

The IABP employed in the PiVAS may be composed of a biocompatible, non-thrombogenic elastomeric material (e.g., Biospan^{®}-S) or other suitable materials and may have features described in U.S. Patent No. 8,066,628, the disclosure of which is incorporated hereby by reference in its entirety. In some embodiments, the maximum device displacement volume may be closely matched to the stroke volume of the heart and is a parameter that may be varied to provide effective counterpulsation therapy. The expandable member may have a displacement volume between about 20 ml and about 60 ml. In some embodiments, the displacement volume is about 50 ml. The IABP may have a length between about 15 cm and about 30 cm.

The expandable member may be implanted via the femoral artery and explanted via the axillary/subclavian artery without the need to enter the chest. Regarding insertion, it may be desirable to use an introducer sheath at both the femoral and axillary/subclavian arteries. An elongated delivery dilator may be disposed in the vasculature between the two introducer sheathes (e.g., by establishing a guidewire rail and then advancing the elongated delivery dilator over the guidewire and subsequently removing the guidewire). The expandable member may be introduced into the femoral artery at the associated introducer sheath. In particular, the distal end of the elongated delivery dilator may be removably coupled to a proximal end of the driveline and the proximal end of the elongated delivery dilator may be pulled to move the expandable member into position. The elongated delivery dilator may be externalized from the axillary/subclavian artery and disconnected from the proximal end of the driveline, which may be also externalized. During introduction of the expandable member into the introducer sheath at the femoral artery, the expandable member may be folded or rolled prior to insertion. A delivery sheath (e.g., a funnel or folding tube) may be utilized to reduce a dimension of the expandable member prior to insertion. The blood pump may be non-obstructive and may lay at least substantially flat in the descending aorta without folds or crevices when deflated. This enables the device to be turned off for prolonged periods. Patients may routinely stop the device for 60-plus minutes with no adverse consequences. The blood pump has been demonstrated to have a durability for over 2.5 years of use.

The driveline may be a thin (e.g., 4.2 mm outer diameter) driveline that shuttles a working fluid (e.g., air, gas, etc.) between the blood pump and the drive unit. The driveline may include an inner driveline and an outer driveline. The inner driveline may be an elongated support structure having a lumen extending there through for delivering working fluid to and from the expandable member. The inner driveline may be positioned at least partially within the patient's vasculature (e.g., between the aorta and the axillary/subclavian artery). After implantation, the inner driveline may have a distal end portion coupled to the expandable member and positioned within the patient's vasculature (e.g., the descending aorta) and a proximal end portion coupled to a distal end of the outer driveline and positioned external to the patient's vasculature at an arteriotomy in, for example, the axillary/subclavian artery, or other suitable blood vessel. The proximal end portion of the inner driveline may be coupled to a distal end of the outer driveline using any suitable technique (e.g., compression rings, suturing, gluing, stitching, etc.). An arterial interface device or stopper device ("AID") may be used to provide hemostasis at an arteriotomy where the inner driveline exists the vasculature. The AID may enable long-term implant using minimally invasive surgical techniques. The AID may include one or more anchoring elements used to secure the device in a desired orientation or position, and one or more ports. For example, one port might provide wire axis to the patient's vasculature. The AID may have features similar to those described in U.S. Patent No. 7,892,162, the disclosure of which is incorporated herein by reference in its entirety. The AID may be deployed or advanced over the inner driveline (e.g., that portion that is externalized from the vasculature) through a shaft in the AID that defines a lumen. The inner driveline may be inserted into and extend through the shaft. The outer driveline may be coupled to the inner driveline at a position proximate the AID.

The outer driveline may also be an elongated support structure having a lumen extending there through. The outer driveline may be positioned at least partially subcutaneously but external to the patient's vasculature. After implantation, the proximal end portion of the outer driveline may be coupled to a skin interface device (described below). The lumen of the driveline (e.g., inner and outer drivelines) may be coupled to the lumen of the expandable member to transport the working fluid to and from the expandable member. The relatively small diameter of the driveline compared to the relatively large axillary/subclavian artery mitigates risk of limb ischemia.

The drive unit may be a small, portable device that actuates the balloon pump by generating a flow of working fluid (e.g., a gas or other fluid such as ambient air or helium) into and out of the expandable member via the driveline. For example, the drive unit may generate a positive pressure to accelerate the working fluid into the expandable member, thereby inflating the expandable member and may induce a negative pressure to withdraw the working fluid from the expandable member, thereby deflating expandable member. The drive unit may utilize a bellows, a blower, a compressor, an accelerator, or other similar features to direct the flow of working fluid into and out of the expandable member. The drive unit may have a feature to prevent over-inflation of the balloon.

The skin interface device ("SID") may be a transcutaneous device that enables the drive unit to drive operation of the expandable member. The SID may provide a stable and/or secure exit site for the driveline (e.g., the outer driveline). In an embodiment where the driveline comprises an inner driveline and outer driveline, the proximal end of the outer driveline may be coupled to an internal facing portion of SID and the drive unit may be coupled to an external facing portion of the SID. As such, SID may direct gases received from the drive unit to the outer driveline for delivery to the expandable member. The SID may be similar to the interface devices described in U.S. Patent No. 10,137,230, the disclosure of which is incorporated herein by reference in its entirety.

The IABP may be triggered, at least in part, by pressure sensor data (e.g., from a pressure sensor element located at the distal tip of the IABP) and/or the patient's ECG via surface ECG sensors. The associated ECG sensors may be coupled to the SID via ECG leads, which relay the measurements received from the sensors to the drive unit via a wired or wireless connection. In other embodiments, the ECG sensors may be wirelessly connected to the drive unit and may transmit the sensed measurements directly to the drive unit without using the SID. The ECG sensors may be implanted, external or both implanted and external. For example, the ECG sensors may be implanted bipolar electrodes positioned at and/or proximate the heart or other appropriate tissue to determine, for example, when the left ventricle is contracting or relaxing. Counterpulsation therapy may be achieved by rapidly inflating the balloon in the aorta immediately after aortic valve closure (dicrotic notch) and rapidly deflating the balloon just before the onset of systole using a drive unit. The dicrotic notch may be sensed by the pressure sensor, and the onset of systole may be predicted or sensed using the ECG signals.

The rapid inflation of the balloon may increase the diastolic aortic pressure, improving end-organ perfusion and coronary perfusion. The rapid deflation of the balloon reduces the ejection pressure of the native ventricle, reducing afterload and left ventricular external work. This embodiment of the PiVAS provides counterpulsation therapy in patients that is more effective than a 40-ml IABP device due to a larger displacement volume. The PiVAS has enhanced durability, has a reduced or eliminated risk of being thrombogenic and/or obstructive, and is overall a low-cost, and less invasive device implant/explant procedure without the need to enter the chest. The PiVAS also has low serious adverse event (AE) burden, and enables non-obligatory support to a less-sick heart failure population (the device may be 'on or off' as needed).

Whether a femoral technique or an axillary technique (e.g., surgical or PiVAS) is used, it is often desirable to reduce the thickness of the IABP while retaining a desired displacement volume (e.g., 50 ml). As balloon thickness is decreased, the IABP may be more readily disposed into the applicable artery (e.g., with respect to PiVAS, through an introducer sheath or delivery sheath), and injury to blood vessel is minimized due to a smaller arteriotomy. Further, at least with balloons manufactured via blow molding, balloon thickness may be at least in part dictated by manufacturing requirements. In particular, such an IABP may lack the structural integrity to remain "vertical" within the descending aorta and prone to kinking, and further prone to roll or fold onto itself (e.g., along either or both of its longitudinal and transverse axes or at one or more angles with respect to either or both axes) or otherwise develop crevices throughout, especially when the balloon is partially deflated during counterpulsation or when the system providing counterpulsation is turned off. The balloon may also kink cyclically, or flip due to the buoyancy when the balloon is inflated in the blood, reducing the durability of the balloon and increasing the likelihood of balloon failure. Balloon failure may also lead to the leaking of air/helium into the blood stream which may cause a stroke, death, or end organ ischemia. Furthermore, balloon failure necessitates the removal and exchange of the balloon which increases cost, procedural risks including vascular injury, bleeding, and mortality. And when an IABP rolls or folds upon itself or otherwise develops crevices when disposed in the descending aorta when fully or inflated, the IABP may become obstructive, which may increase cardiac workload leading to increased heart failure or death and may require an emergency surgical procedure to remove the balloon.

Accordingly, there exists a need to prevent IABPs from rolling or folding upon themselves (or otherwise developing crevices) when disposed in a descending aorta.

### BRIEF DESCRIPTION OF THE DRAWINGS

Many aspects of the present technology may be better understood with reference to the following drawings. The components in the drawings are not necessarily to scale, and instead emphasis is placed on illustrating clearly the principles of the present disclosure.
FIGs. 1A and 1B illustrate an IABP disposed in the descending aorta using two different techniques: a femoral technique (FIG. 1A) and an axillary technique (FIG. 1B).
FIG. 2 illustrates side views of a support structure configured to be removably disposed in a blood pump assembly, the support structure having a first type of nipple tip in accordance with an embodiment of the present disclosure.
FIG. 3 illustrates implantation of the support structure of FIG. 2 comprising an elastic or superelastic material into an exemplary blood pump assembly comprising a balloon and a driveline in accordance with an embodiment of the present disclosure.
FIG. 4 illustrates implantation of the support structure of FIG. 2 comprising a shape memory or pseudoelastic material into an exemplary blood pump assembly comprising a balloon and a driveline in accordance with an embodiment of the present disclosure.
FIG. 5 illustrates explantation of the support structure of FIG. 3 or FIG. 4 from an exemplary blood pump assembly comprising a balloon and a driveline in accordance with an embodiment of the present disclosure.
FIG. 6 illustrates a connector in accordance with a first embodiment of the present disclosure.
FIG. 7 illustrates a connector in accordance with a second embodiment of the present disclosure.
FIG. 8 illustrates installation of the connector of FIG. 6 onto the proximal end of the driveline of the support structure of FIG. 3, in accordance with an embodiment of the present disclosure.
FIG. 9 illustrates installation of the connector of FIG. 7 onto the proximal end of the driveline of the support structure of FIG. 4, in accordance with an embodiment of the present disclosure.
FIG. 10 illustrates side views of a support structure configured to be removably disposed into a blood pump assembly, the support structure having a second type of nipple tip in accordance with an embodiment of the present disclosure.
FIG. 11 illustrates exemplary placements of radiopaque materials on the support structures of FIGs. 2 and 10.
FIG. 12 illustrates use of a buddy guidewire during implantation of the support structure of FIG. 3 into the blood pump assembly of FIG. 3 in accordance with an embodiment of the present disclosure.
FIG. 13 illustrates use of a buddy guidewire during implantation of the support structure of FIG. 4 into a blood pump assembly of FIG. 4 in accordance with an embodiment of the present disclosure
FIG. 14 illustrates use of a buddy guidewire during explantation of the support structure of FIG. 3 or FIG. 4 from the blood pump assembly of FIG. 3 or Fig. 4 in accordance with an embodiment of the present disclosure.
FIG. 15 illustrates a side view of a support structure configured to be removably disposed in a blood pump assembly, the support structure having head region with a flexible tip portion and a non-flexible body portion in accordance with an embodiment of the present disclosure.
FIG. 16 illustrates implantation of the support structure of FIG. 15 into a blood pump assembly comprising a balloon and a driveline in accordance with an embodiment of the present disclosure.
FIG. 17 illustrates explantation of the support structure of FIG. 16 from the blood pump assembly of FIG. 16 in accordance with an embodiment of the present disclosure.
FIG. 18 illustrates the support structure of FIG. 15 disposed within a blood pump assembly comprising a balloon and a driveline, and further illustrates a connector coupled to the proximal end of the driveline, in accordance with an embodiment of the present disclosure.
FIG. 19 illustrates a side view of a support structure configured to be removably disposed in a blood pump assembly, the support structure having head region with a blunted tip in accordance with an embodiment of the present disclosure.
FIG. 20 illustrates implantation of the support structure of FIG. 19 into a blood pump assembly comprising a balloon and a driveline in accordance with an embodiment of the present disclosure.
FIG. 21 illustrates explantation of the support structure of FIG. 20 from a blood pump assembly of FIG. 20 in accordance with an embodiment of the present disclosure.
FIG. 22 illustrates the support structure of FIG. 19 disposed within a blood pump assembly comprising a balloon and a driveline, and further illustrates a connector coupled to the proximal end of the driveline, in accordance with an embodiment of the present disclosure.
FIG. 23 illustrates an arterial interface of stopper device providing hemostasis at the arteriotomy site associated with a balloon implanted using an axillary/subclavian implantation technique.
FIG. 24 illustrates a blood pump support method in accordance with one or more embodiments of the present disclosure.

### DETAILED DESCRIPTION

NuPulseCV has developed: (1) an IABP assembly with one or more sensors (the "Sensor Technology"), generally described in a U.S. Patent Application filed on the same date as the present disclosure, entitled "Intra-Aortic Balloon Pump Assembly with Pressure Sensor," identifying Joshua Ryan Woolley, Sonna Manubhai Patel-Raman, Guruprasad Anapathur Giridharan as inventors, and having attorney-docket number 8018US00/236533-30034; and (2) an intra-aortic balloon pump assembly with a balloon configured for greater support, improved reinforcements, new markers, and other improvements (the "Improved Balloon Pump Assembly Technology"), generally described in a U.S. Patent Application filed on the same date as the present disclosure, entitled "Intra-Aortic Balloon Pump Assembly," identifying Joshua Ryan Woolley, Robert Christopher Hall, Duane Sidney Pinto, and Guruprasad Anapathur Giridharan as inventors, and having attorney-docket number 8019.US00/236533-30037, the contents of both applications are incorporated by reference herein in their entirety. The Sensor Technology solves certain problems associated with, among other things, axillary implantation of a IABP assembly using a pressure sensor disposed at the tip (or distal end) of the IABP, and the Improved Balloon Pump Assembly Technology solves certain problems associated with, among other things, the design of current IABPs and their drivelines. As described in the above-disclosed and incorporated U.S. Patent Application directed to Improved Balloon Pump Assembly Technology, it may be desirable for a balloon to have a wall thickness of between approximately 0.025-0.035 inches (0.635-0.889 mm), between approximately 0.010-0.014 inches (0.254-0.356 mm) between approximately 0.003-0.012 inches (0.076-0.305 mm) or between approximately 0.003-0.016 inches (0.076-0.406 mm). While an IABP with a wall thickness of between approximately 0.025-0.035 inches, 0.010-0.014 inches, 0.003-0.012 inches, or 0.003-0.016 inches may facilitate entry into the femoral or axillary/subclavian arteries, such an IABP with relatively thin walls render it susceptible to a lack of structural integrity. As explained herein, the present disclosure solves various problems associated with using thin-walled IABPs for mechanical circulatory support devices (e.g., counterpulsation).

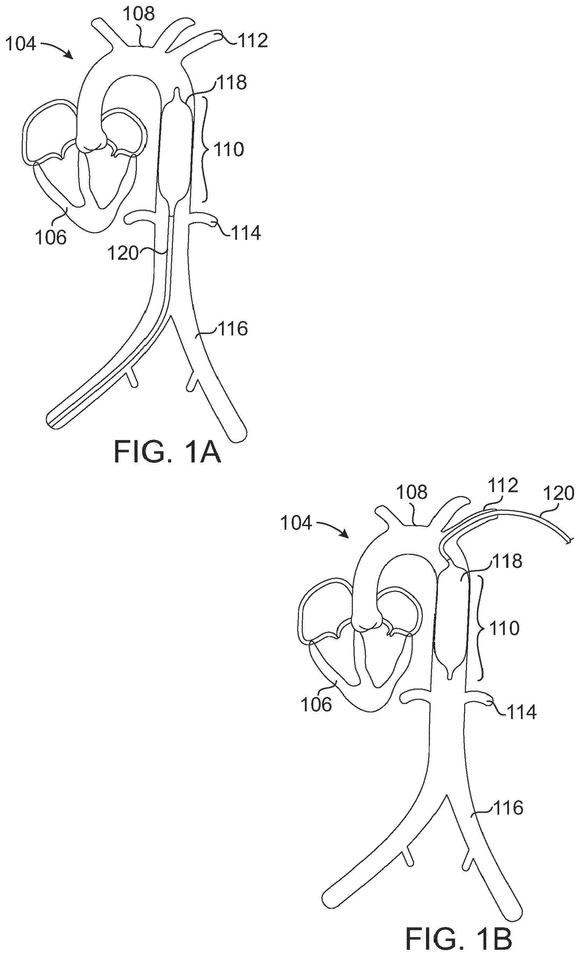

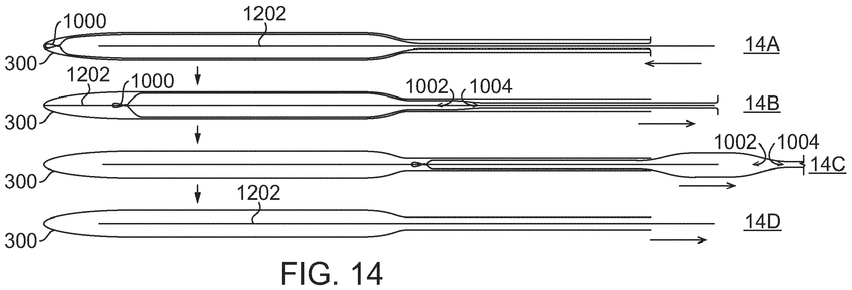

Specific details of several embodiments of the present technology are described herein with reference to FIGs. 1-24. Other applications and other embodiments in addition to those described herein are within the scope of the present technology. It should be noted that other embodiments in addition to those disclosed herein are within the scope of the present technology. Moreover, a person of ordinary skill in the art will understand that embodiments of the present technology may have configurations, components, and/or procedures in addition to those shown or described herein and that these and other embodiments may be without several of the configurations, components, and/or procedures shown or described herein without deviating from the present technology. Reference throughout this description to "one embodiment," "an embodiment," "one or more embodiments," an "nth embodiment," or "some embodiments" means that a particular feature, support structure or characteristic described in connection with the embodiment is included in at least one embodiment of the present disclosure. Thus, use of such terminology is not necessarily referring to the same embodiment. For example, it is expressly contemplated that the features described herein may be combined in any suitable manner in one or more embodiments.

FIGs. 1A and 1B illustrate an IABP 118 disposed in the aortic arch vasculature 104, and more particularly in the descending aorta 110, using two different techniques: a femoral technique (FIG. 1A) and an axillary technique (FIG. 1B). The aortic arch vasculature 104 includes the heart 106, the aortic arch 108, the axillary/subclavian artery 112, the descending aorta 110, the two branches of the renal artery 114 and the two branches of the common iliac artery 116. In the femoral implantation of FIG. 1A, the driveline 120 extends through the femoral artery (not depicted). In the axillary implantation of FIG. 1B, the driveline 120 extends through the axillary or subclavian artery 112. IABP 118 in each of FIGs. 1A and 1B are not drawn to scale. As described in the above-disclosed incorporated U.S. Patent Application directed to Improved Balloon Pump Assembly Technology, IABP 118 may extend caudally past the renal artery 114.

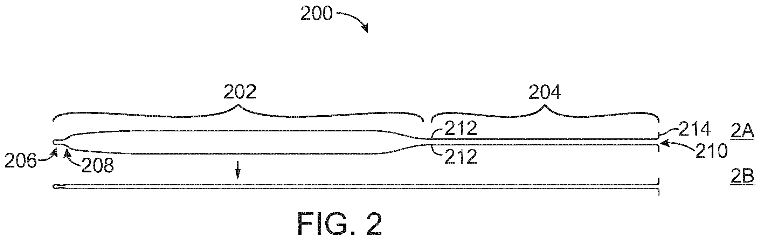

FIG. 2 illustrates side views 2A, 2B of an exemplary blood pump support apparatus in the form of a support structure 200 configured to be disposed in a blood pump assembly (not depicted) in accordance with an embodiment of the present disclosure. The exemplary support structure 200 has a head region 202 and a tail region 204. The profile of head region 202 may taper distally toward the distal end of the head region 202 to form a nipple 206 having a base 208 defined as a pinch point associated with the tapering of the head region 202.

Structure 200 may be made of wire or any wire-like material. The exemplary support structure 200 is configured in the shape of a loop having an open portion 210 disposed at the proximal end of the tail region 204. In one embodiment, the tail region 204 has two stem ends 212 and the head region 202 is a contiguous open-ended loop with two proximal ends. In one embodiment, the stem ends 212 are substantially parallel. Substantially parallel may mean that each stem end 212 does not deviate more than 10 degrees from a longitudinal axis of the exemplary support structure 200. Each distal end of the stem ends 212 is contiguous with a respective proximal end of the contiguous head region 202, and the proximal ends of the stems ends 212 are open defining open portion 210. In one embodiment, the proximal ends of the stem ends 212 flare outward and form flanges 214. In one embodiment the flanges 214 are substantially aligned with the transverse axis of the support structure 200. In this embodiment, substantially aligned means within 10 degrees of the reference transvers axis. In other embodiment, the flanges are not substantially aligned with the transverse axis. In one embodiment flanges 214 are flared at an angle with respect to the transverse axis. In one embodiment, the shape of the loop may be dagger-shaped. In another embodiment, the shape of the loop may substantially conform to the shape of a balloon associated with the blood pump assembly into which it is designed to be disposed within. A shape substantially conforms to the shape of the balloon means that the angles or curvatures deviate less than 10% of values of such angles and/or curvature associated with the inside walls of the balloon. Other shapes may be utilized.

Nipple 206 may be formed by tapering opposing sides of the loop-shaped head region. The opposing sides of head region may be touchingly adjacent at base 208 or not touching adjacent at base 208. The latter is depicted in FIG. 2.

Head region 202 may be comprised of a material that is the same or different than tail region 204. Head region 202 and tail region 204 may comprise a material that exhibits elasticity or superelasticity or a material that exhibits shape memory (also known as pseudoelasticity). Elastic and superelastic materials may have a resting state and a loaded state. When in an initial resting state, the material may take on a shape that is determined during manufacture. An elastic or superelastic material may transition to a loaded state in the presence of an applied mechanical load that is equal to or greater than a threshold amount. If an applied mechanical load does not equal or is not greater than the threshold amount, the material will remain in its resting state. For so long as a mechanical load is applied that equals or is greater than a threshold amount, the elastic or superelastic material may be in a loaded state and may take on one or more different shapes based on the extent of the mechanical loading. That is, the shape of the material in the presence of a first mechanical load may be different than the shape of the material in the presence of a second mechanical loading. When in a loaded state, the structure 200 can be said to be deformed and have a deformed shape.

If all applied mechanical loads are removed or if the then-applied mechanical loads are not at least as great as the threshold amount, the material will transition to a resting state. The shape of the resting state after transition from a loaded state will be the same as determined during manufacture provided that the previously applied mechanical load does not exceed a material-specific load amount that causes nominal permanent deformation of the material (the "nominal deformation load amount"). If the previously applied mechanical load exceeds the material's nominal deformation load amount but does not exceed a material-specific load amount that causes significant permanent deformation (the "significant deformation load amount"), then the material in its subsequent resting shape will return to a shape that substantially conforms to the shape determined during manufacture. A shape substantially conforms to its shape determined during manufacture when angles or curvatures deviate less than 10% of values of such angles and/or curvature established during manufacture. If the previously applied mechanical load exceeds the significant deformation load amount, then the material in its subsequent resting shape will either retain the shape during the loaded state (i.e., it will not take on a new shape) or it will return to a shape that does not substantially conform to the shape determined during manufacture (i.e., the value of angles and curvatures may vary by more than 10% as compared to values of such angles and/or curvatures established during manufacture).

An elastic material is different from a superelastic material in that its nominal deformation load amount and its significant deformation load amount may be less that the corresponding amounts associated with a superelastic material. An example of a material that exhibits elasticity is stainless steel. An example of a material that exhibits superelasticity is Nitinol, provided that the Nitinol is not at a temperature below its memory transfer temperature (as described below).

Shape memory materials may also have a resting state and a loaded state. When in an initial resting state, the material may take on a shape that is determined during manufacture. A shape memory material may transition to a loaded state when cooled to a temperature within a material-specific deformation temperature range (e.g., 0-4 degrees C for some compositions of Nitinol) and while such shape memory material remains at the deformation temperature range, a mechanical load is applied that is equal to or greater than a threshold amount. If an applied mechanical load is not equal to or is not greater than the threshold amount, the material remains in its resting state at least in respect of its shape. But if an applied mechanical load is at or exceeds the threshold amount when the temperature of the shape memory material is within the deformation temperature range, then the applied load will cause the shape memory material to take on one or more different shapes (i.e., be deformed) based on the extent of the mechanical load. That is, the shape of the shape memory material in the presence of a first mechanical load may be different than the shape of the material in the presence of a second mechanical load, in each instance for so long as the shape memory material is within the deformation temperature range. When in a loaded state, the structure 200 can be said to be deformed with a deformed shape.

As the temperature of the shape memory material exceeds the deformation temperature range but remains below the material-specific memory transfer temperature range (or the lowest temperature within that range), the shape memory material will retain the deformed shape caused by the applied mechanical load when the shape memory material was within the deformation temperature range. That is, the shape memory material will remain in a loaded state. However, as the shape memory material is heated to a temperature that is at or exceeds its memory transfer temperature range (e.g., 30-34 C for Nitinol), the shape memory material will transition to a resting state and to the shape as determined during manufacture, provided, that the previously applied mechanical load during deformation does not exceed the material-specific nominal deformation load amount. If the previously applied mechanical loading during deformation exceeded the nominal deformation load amount but does not exceed the shape memory material's significant deformation load amount, then the shape memory material in its subsequent resting state will return to a shape that substantially conforms to the shape determined during manufacture. A shape substantially conforms to its shape determined during manufacture when angles or curvatures deviate less than 10% of values of such angles and/or curvature established during manufacture. If the previously applied mechanical load during deformation exceeded the significant deformation load amount, then the material in its subsequent resting shape will either retain the deformation shape during the loaded state (i.e., it will not take on a new shape) or it will return to a shape that does not substantially conform to the shape determined during manufacture (i.e., the value of angles and curvatures may vary by more than 10% as compared to values of such angles and/or curvatures established during manufacture).

Returning to FIG. 2, support structure 200 at 2A is in an exemplary resting state (e.g., as determined by manufacture), while support structure 200 at 2B is in an exemplary loaded state (e.g., a loaded state where the applied mechanical load that caused the deformed shape does not exceed the nominal deformation load amount).

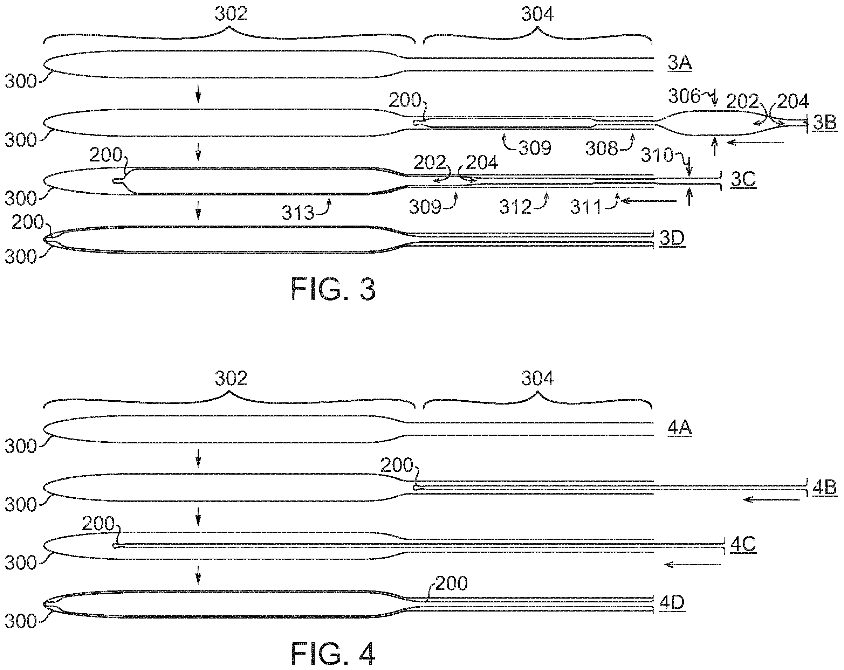

The support structure 200 is configured to be disposed within a blood pump assembly having a balloon and driveline. A width of the deflated balloon may be greater than the interior diameter of the driveline. The head region 202 may be shaped and sized such that when in its resting state (e.g., as depicted in 2A) and with reference to a deflated balloon or a substantially deflated balloon (e.g., balloon 302, illustrated in Fig. 3 at 3A), the head region 202 takes on the shape and size of the planar circumference of the interior wall of the deflated or substantially deflated balloon 302. In other embodiments, the head region 202 takes on the shape of the planar circumference of the interior wall of the deflated or substantially deflated balloon 302, but is of a smaller size. Other shapes are expressly contemplated. The width of the profile of the head region 202 in such a resting state may be greater than the interior diameter of the driveline (e.g., driveline 304, illustrated in FIG. 3 at 3B). Thus, the shape and/or size of the head region 202 when disposed entirely in a balloon 302 may prevent or oppose the head region 202 from backing out of the balloon 302 without the application of an external extraction force at the tail region 204 sufficient to collapse the exemplary support structure 200 (discussed below). In one embodiment, the distance between the two stems ends 212 of the tail region 204 when in a resting state is equal to, greater than, or less than the interior diameter of the driveline 304.

FIG. 3 at 3A illustrates an exemplary blood pump assembly 300 having balloon 302 and driveline 304. Blood pump assembly 300 may be as described above with respect to PiVAS. That is, it may comprise the same or similar materials, have the same or similar sizes, and be disposed in the descending aorta using the same or similar techniques. The width of the profile of the deflated balloon 302 may be greater than the interior diameter of the driveline 304. Balloon 302 is disposed at a distal region of the blood pump assembly 300 and driveline 304 is disposed at a proximal region of the blood pump assembly 300. Blood pump assembly 300 may be used to provide mechanical circulatory support such as counterpulsation.

FIG. 3 at 3B-3D illustrates an exemplary implantation of an exemplary support structure 200 comprising elastic or superelastic material into exemplary blood pump assembly 300. In particular, head region 202 is configured to be advanced through driveline 304 such that head region 202 is removably disposed within balloon 202 and tail region 204 is removably disposed within the driveline 304. With initial reference to FIG. 3 at 3B, a mechanical load 306 (up and down arrows) may be applied by hand (e.g., by a medical professional) to head region 202 at a location external to the proximal end of driveline 304 to advance support structure 200 into the blood pump assembly 300. In addition to load 306, an advancement force may be applied to push or advance support structure 200 into assembly 300 in the direction depicted. The applied mechanical load 306 may cause at least a portion 308 of support structure 200, and in particular head region 202, to assume a loaded shape that resembles the loaded shape illustrated in FIG. 2 at 2B. Once inside driveline 304, the support structure 200, and in particular head region 202, is no longer subject to the mechanical load 306, but may be subject to a mechanical load applied by the inside walls of driveline 304. This driveline load may cause at least another portion 309 of support structure 300, and in particular head region 302, to assume a different loaded shape associated with the driveline mechanical load. As is noted above, a loaded shape may be a deformed shape.

With reference to FIG. 3 at 3C, the head region 202 has been advanced into the assembly, such that a distal end of the head region 202 is disposed in the balloon 302 and a proximal end of the head region 202 is disposed in driveline 304 with a portion of the tail region 204 disposed in the driveline 304 and a portion of the tail region 204 externalized from the proximal end of driveline 304. The distal end of head region 202 may be exposed to either no mechanical load or a nominal mechanical load such that this portion 313 of head region 202 takes on its resting shape (e.g., as generally depicted in FIG. 2 at 2A). The proximal end of head region 202 that remains disposed in the driveline 304 may be subject to the driveline mechanical load and may define portion 309 of support structure that assumes the loaded shape associated with the driveline mechanical load at this stage of implantation.

Another mechanical load 310 (up and down arrows) may be applied by hand (e.g., by a medical professional) to externalized portion of tail region 204 at a location immediately external to the proximal end of driveline 304 to continue to advance support structure 200 into the blood pump assembly 300. A continued advancement force to advance support structure 200 into assembly 300 may be applied in the direction illustrated. The applied mechanical load 310 may cause at least a portion 311 of support structure 200, and in particular tail region 204, to assume a loaded shape that resembles the loaded shape illustrated in FIG. 2 at 2B. Once inside driveline 304, the support structure 200, and in particular tail region 204, may no longer be subject to the mechanical load 310, but may be subject to a mechanical load applied by the inside walls of driveline 304, causing at least another portion 312 to take on either a loaded shape (if driveline exerts a force on tail region 204) or to take on a resting shape (if driveline does not exert a force on tail region 204). In the illustrated example, there is no mechanical load created by driveline 304 at portion 312 and the shape of the tail region 204 at portion 312 is the resting shape, e.g., as is generally depicted at FIG. 2 at 2A.

With reference to FIG. 3 at 3D, support structure 200 is fully disposed within assembly 300 and the entirety of support structure 200 is illustrated as being in its resting state. As was suggested above, it is contemplated that mechanical loads could be respectively applied by balloon 302 and/or driveline 304 to head region 202 and tail region 204 to cause a slightly loaded shape. Proximal ends of tail region 204 may be sized such that when support structure 200 is fully disposed within assembly 300, proximal ends terminate at or within a predetermined distance of proximal end of driveline 304. Alternatively, proximal ends of tail region 204 could be cut to size in the field. Flanges 214 may be in contact with proximal ends of driveline 304. Flanges 214 may operate to prevent tail region from advancing further within driveline 304. Flanges 214 may operate to prevent distal tip 206 from being in touching contact with the inside wall of the distal end of balloon 302. Finally, as foretold above, the proximal end of head region 202 may be shaped to resist or oppose mechanical loads applied during operation (e.g., during counterpulsation of balloon 302) which may otherwise act to force head region out of balloon 302. In particular, the shape of the proximal end of balloon 302 may be shaped such that the anticipated operational mechanical loads are insufficient to place support structure 200 into a loaded state to explant support structure 200.

A lubricant which reduces friction (e.g., hydrophilic and hydrophobic lubricants like NuSil silicone lubricants, Surgilube, Viperslide etc.) may be used to facilitate advancement of the support structure 200 into the blood pump assembly 300. Balloon 302 may be partially inflated (e.g., at 50%) to facilitate advancement of the support structure 200 into the blood pump assembly 300. Once the support structure 200 is fully disposed within assembly 300, balloon 302 may be inflated and/or cyclically inflated/deflated for purposes of counterpulsation.

FIG. 4 illustrates an exemplary implantation of an exemplary support structure 200 comprising shape memory material into exemplary blood pump assembly 300. FIG. 4 at 4A is identical to FIG. 3 at 3A. The process may start by placing support structure 200 into a loaded state as depicted in FIG. 2 at 2B. Ideally, the support structure 200 will retain its loaded shape throughout the implantation process. Accordingly, no mechanical loads would need to be applied to maintain a loaded shape, and the support structure 200 is merely advanced into assembly 300 as depicted in 4B-4D by application of an advancement force. FIG. 4 at 4D is identical to FIG. 3 at 3D. However, because assembly 300 may be located within the vasculature of a human body during implantation, the internal temperature of the human body may cause support structure 200 to be heated to a temperature at or in excess of its memory transfer temperature. If support structure 200 (or a portion thereof) is heated to its memory transfer temperature, it will transition to its resting shape and one or more mechanical loads may need to be applied to advance support structure 200 into assembly 300, as is generally described with respect to FIG. 3.

As described above in connection with FIG. 3, a lubricant may be used during implantation and balloon 302 may be partially inflated to facilitate advancement. Once the support structure 200 is fully disposed within assembly 300, balloon 302 may be inflated and/or cyclically inflated/deflated for purposes of counterpulsation.

Although not depicted, implementation of support structure 200 into assembly 300, regardless of material type, may be facilitated by implantation of support structure 200 into a delivery sleeve (not depicted). Once inserted into a delivery sleeve, the delivery sleeve along with the support structure 200 therein may be implanted into assembly 300 and then the sleeve may be explanted, leaving support structure 200 disposed within assembly 300. A lubricant may be used on the inside and/or outside of delivery sleeve to facilitate the implant of the support structure 200 into delivery sleeve, the implant of the delivery sleeve into assembly 300 and the explant of delivery sleeve from support structure. The delivery sleeve may apply a sufficient mechanical load to retain support structure 200 in a sufficiently loaded state for implantation.

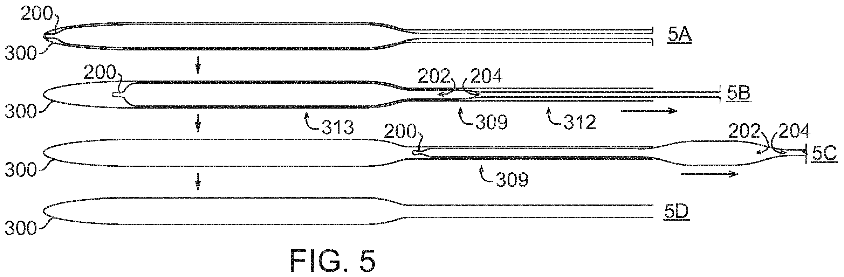

FIG. 5 illustrates an exemplary extraction 5A-5D of support structure 200 of FIG. 2 from a blood pump assembly 300 of FIG. 3. The depiction is applicable to support structures 200 that are comprised of elastic or superelastic materials and support structures that are comprised of shape memory materials. In some instances, support structure may be comprised of a material such as Nitinol that may exhibit both shape memory and superelasticity. An external extraction force may be applied at the proximal end of the tail region 204, e.g., from a medical professional, at FIG. 5 at 5B and 5C. As the external extraction force is increased, the support structure 200 (i.e., in particular the head region 202) may collapse into the driveline 302, thus beginning a process that is the opposite of what was described in FIG. 3 as it relates to insertion. As is depicted in FIG. 5 at 5B and 5C, a driveline mechanical force may be applied during explantation, just as one was applied during implantation, causing portion of head region 309 to take on a loaded state and shape when disposed in driveline 304. Portion 312 of tail region 204 may be subject to some or no driveline mechanical loading. In one embodiment, a delivery sleeve (not depicted) may be first inserted into assembly 300 over support structure 200 (e.g., using a lubricant) prior to application of an external extraction force on tail region 204.

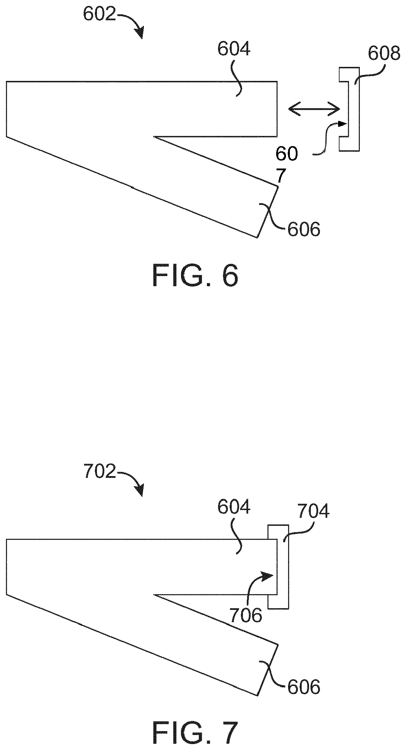

FIG. 6 illustrates a blood pump support apparatus in the form of a connector 602 in accordance with a one embodiment of the present disclosure. Connector 602 may include anti-backout port 604 and a removable cap 608 capable of being removably coupled to the proximal end of the anti-backout port 604. Distal end of anti-backout port 604 is configured to be installed on (e.g., coupled to or otherwise secured to) proximal end of driveline 304. In one embodiment, distal end of connector 602 is configured to clamp on to proximal end of driveline 304 such that an air tight connection seal is formed. In other embodiments, distal end of connector 602 is secured to the proximal end of driveline 304 using a threaded connection, a house barb connection, a tuohy borst valve or any suitable connection or fastening technique. Anti-backout port 603 may be configured to receive a portion of tail region 204 (e.g., the proximal end(s) of the tail region 204) when connector 602 is installed on proximal end of driveline 304.

Removable cap 608 and proximal end of anti-backout port 604 may have complementary threaded surfaces (not depicted) to permit the removable coupling of cap 608 to port 604. Other means of mechanically coupling cap 608 to port 604 are contemplated such as various locks or locking mechanisms.

Connector 602 may include a clamp (not depicted) that clamps onto or locks and thereby secures the tail region 204 (e.g., at proximal end(s) of tail region 204). In an alternate embodiment, inside surface 607 of cap 608 may contain the proximal end(s) of tail region 204, thereby securing the tail region 204. In such an embodiment, the proximal end(s) of tail region do not extend past the cap 608. In one embodiment, proximal ends of tail region 204 are sized to rest on or proximate to inside surface 607 of cap 704. In an embodiment where proximal ends of tail region 204 have flanges 214, support 200 may be configured such that the flanges 214 rest on or proximate to the inside surface 607 of cap 704. By securing the tail region 204, whether through a clamping, locking, containing, or otherwise, support structure 200 itself and its component parts may be secured such that operational forces (e.g., buoyancy) do not drive support structure 200 out of assembly 300 during operation (e.g., during counterpulsation).

Connector 602 may include a pneumatic port 606 configured to provide a fluid connection with a driveline 304 lumen and the inner volume of balloon 302 (e.g., for the purposes of counterpulsation) when connector 602 is coupled to proximal end of driveline 304.. In one embodiment, the proximal end of pneumatic port 606 is configured to be coupleable to the output of a drive unit (not depicted). For example, the proximal end of pneumatic port 606 may be configured as a hose barb connector (not depicted) and a tube that connects to the drive unit may be removably coupled to the hose barb connector such that gases from the drive unit may be used to inflate and deflate balloon 302.

FIG. 7 illustrates a blood pump support apparatus in the form of a connector 702 in accordance with another embodiment of the preset disclosure. Connector 702 may be identical to connector 602 with the exception that cap 704 is fixed to the proximal end of the anti-backout port 604 (e.g., during manufacture) and is not removeable. Inside surface 706 of cap 704 may contain the proximal end(s) of tail region 204, thereby securing the tail region 204, as was described in connection with FIG. 6.

Connectors 602, 702 are illustrated as y-connectors. However, other types of 3-way connectors (e.g., other types of 3-way connectors) may be utilized. For example, anti-backout port 604 may be the same port as pneumatic port 606. Other ports may be included in the same connector 602, 702.

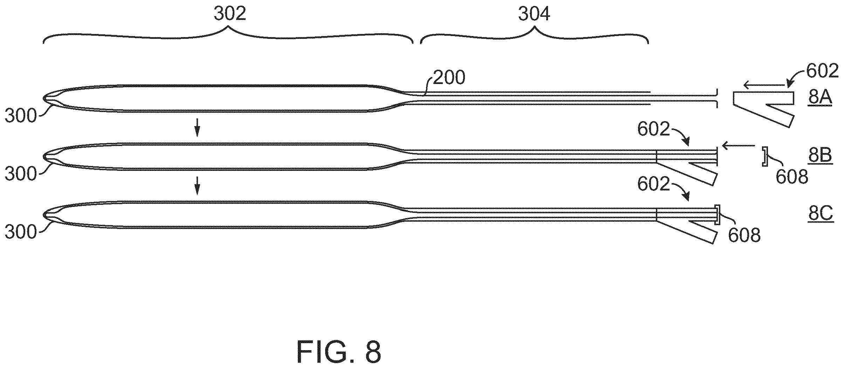

An example of connector 602 being installed on proximal end of driveline 304 is depicted in FIG. 8. In this embodiment, the tail region 202 extends beyond the proximal end of driveline 304 when support structure 200 is fully disposed in assembly 300, as is generally depicted at 8A. Anti-backout port 604 of connector 602 may be slid over the stem ends 212 of tail region 202 such that the flanges 214 are exterior to the proximal end of anti-backout port 604 as is generally depicted in 8A-8B. Distal end of connector 602 is secured to proximal end of driveline in 8B and in 8C proximal end of tail region 202 is clamped and secured while cap 608 is disposed on the proximal end of connector 602. Cap 608 in this embodiment includes the clamp. Clamp, may be disposed elsewhere in connector 602 (e.g., in the anti-backout port 604).

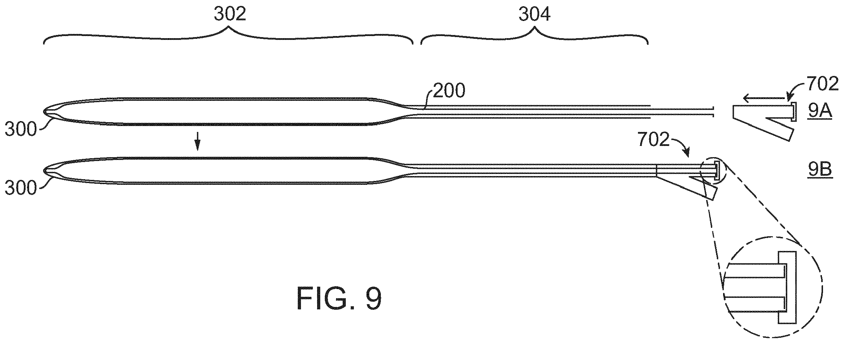

An example of connector 702 being installed on proximal end of driveline 304 is depicted in FIG. 9. The process is generally the same, but cap is fixed on port 604. Once distal end of port 604 is secured to proximal end of driveline 304 in 9A, the proximal ends of tail region 204 are located proximate the interior surface 706 of cap 704 as is depicted in 8B. Here, flanges 214 are located proximate the interior surface 706 of cap 704.

Extraction of support structure 200 from assembly 300 after installation of connector 602, 702 may proceed in a manner substantially similar to that described in connection with FIG. 5, however, before an external extraction force is applied at the proximal end of tail region 204, connector 602, 702 is first uninstalled (i.e., uncoupled or de-secured) from proximal end of driveline 304, or if connector 602, is installed one might also remove cap 608 to access support structure 200. If connector 602, 702 includes a clamp or lock, the tail region 205 may also be released from the clamp or lock. The balloon may be partially or fully inflated to facilitate the extraction of support structure 200.

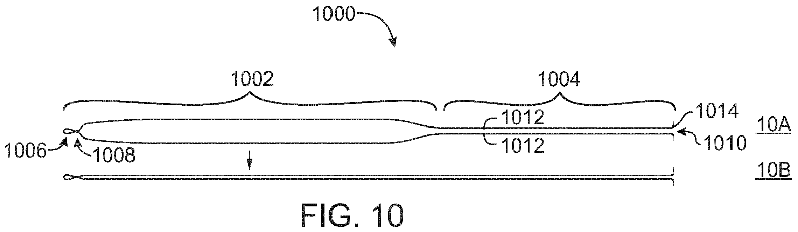

FIG. 10 illustrates side views 10A, 10B of an exemplary blood pump support apparatus in the form of a support structure 1000. Support structure 1000 is substantially identical to FIG. 2, with the exception that support structure 1000 includes a different nipple. Support structure 1000 includes head region 1002 and tail region 1004. The profile of head region 1002 tapers distally toward the distal end of the head region 1002 and the head region 1002 crosses-over itself (e.g., is twisted on itself) at base 1008 to form nipple 1006. Structure 1000 may include stem ends 1012, flanges, 1014 and opening 1010, which may be the same or substantially similar to stems 212, flanges 214 and opening 210. FIG. 10 at 10A depicts support structure 1000 in an exemplary resting state and resting shape. FIG. 10 at 10B depicts support structure 1000 in an exemplary loaded state and loaded shape.

Support structure 1000 can be implanted and explanted into assembly 300 in the same manner as support structure 200 as was generally described in connection with FIGs. 3-5. Once implanted, connectors 602, 702 may be installed on driveline 304 in the same manner as was described in connection with FIGs. 8-9. And extraction of support structure 1000 from an assembly having connector 602, 702 installed thereon may be the same as was described in connection above.

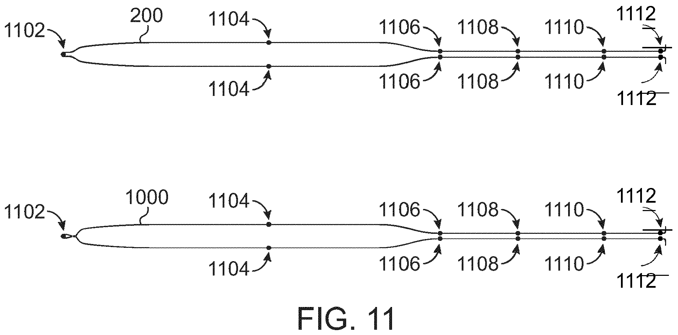

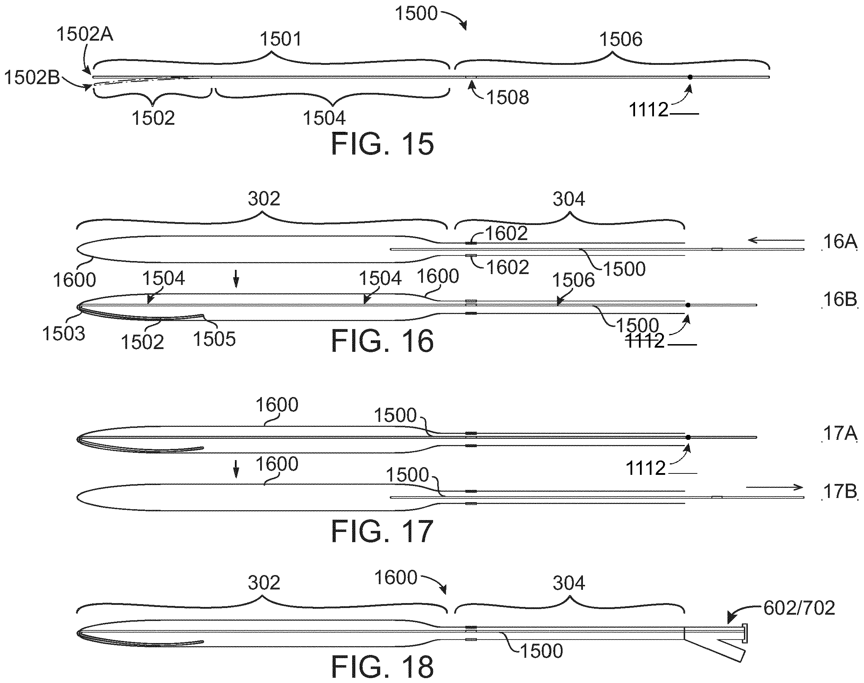

Radiopaque material may be disposed on support structure 200, 1000 to track the position of such structure 200, 1000 during radiological intervention (e.g., x-ray or fluoroscopy) or other imaging techniques. FIG. 11 illustrates exemplary placements of radiopaque materials 1102-1110 on the support structure 200, 1000 of FIGs. 2 and 10. Radiopaque material 1102-1110 may be displayed strategically throughout the body of the support structure 200, 1000 to assist a medical professional ensure proper placement in a patient. Material 1102 may be disposed at the distal end of head region (e.g., near or at the nipple tip 206, 1006); material 1104 may be disposed at a midpoint of head region 202, 1002; material 1106 may be disposed at a proximal end of head region 202, 1002 or at a distal end of the tail region 204, 1004; and material 1108 and 1110 may be disposed at first and second midpoints of the tail region 204, 1004. In one embodiment, radiopaque materials 1104-1110 may be disposed in pairs on opposite sides of support structure 200, 1000, as is depicted. In other embodiments, radiopaque materials 1104-1110 may be disposed on one or the other sides of support structure 200, 1000. In one embodiment, some or all of the radiopaque materials 1102-1110 are deployed on support structure 200, 1000. For example, although FIG. 11 depicts an example where multiple radiopaque materials 1102-1110 are deposited on structures 200, 1000, other examples may use less or more radiopaque materials. Some embodiment, may deploy a single radiopaque material. One or more of such radiopaque materials (e.g., 1102-1110) may be used to align the support structure 200, 1000 during radiological intervention (e.g., x-ray or fluoroscopy) or other imaging techniques. Optionally, complementary radiopaque materials are disposed on the blood pump assembly 300 or portion(s) thereof, for example, on driveline 304 (not depicted in FIGS. 3-5, or 8-9, but generally depicted and described in connection with FIGs. 16-18 as element 1602) to give a visual indicator of proper placement. In another embodiment, complementary radiopaque materials may be disposed on balloon 302. One of skill in the art will recognize that support structure 200, 1000 itself may comprise a radiopaque material and that radiopaque materials 1102-1110 may be additional deposits of radiopaque material.

In another embodiment, non-radiopaque material 1112 may be added to a location on the support structure 200, 1000, for example to a location on the tail region 204, 1004. In one example, the non-radiopaque material is disposed at a proximal end of tail region 204, 1004. The non-radiopaque material 1112 may serve as a visual marker, such that when the non-radiopaque material 1112 is aligned with a predetermined location of driveline 304 (or visible at or proximate a location external to the driveline 304) there is provided an indication that support structure 200, 1000 or the head region 202, 1002 is properly and fully inserted into balloon 302. For example, non-radiopaque material 1112 may be disposed on a location of the tail region 202, 1004 that corresponds to a proximal end of driveline 304 when support structure 200, 1000 is properly and fully inserted and deployed within balloon 302. In another example, the proximal end of driveline 304 may be trimmable at a location at or external to the axillary/subclavian artery 112 such that when the support structure 200, 1000 or the head region 202, 1002 is properly and fully inserted and deployed within balloon 302, the non-radiopaque material 1112 may be located at or proximate the trimmed location (e.g., the non-radiopaque material is aligned with the trimmer location). The non-radiopaque material 1112 may be disposed at any location on structure 200, 1000 or tail region 204, 1004.

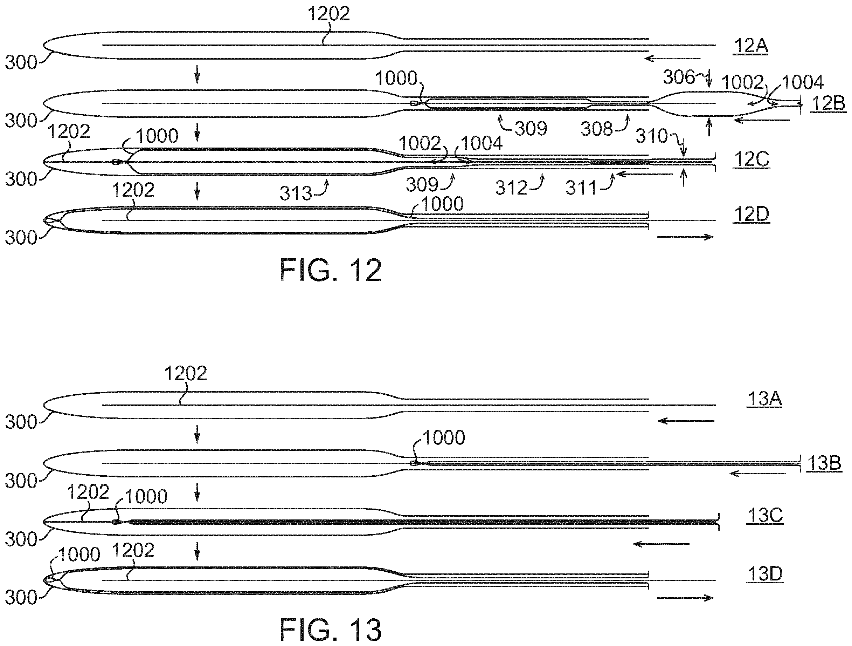

FIGs. 12-14 depict exemplary implantations and an explantation of the support structure 1000 into blood pump assembly 300 using a guidewire 1202 (e.g., a buddy guidewire). Guidewire may have an atraumatic tip. The process may be identical to that described with respect to FIGs. 3-5, but here support structure 1000 is used instead of support structure 200 and a guidewire 1202 is used to facilitate implant and explant of support structure 1000. The implantation and explantation process described below equally applies to use of guidewire 1202 to implant and explant support structure 200. Regarding the use of guidewire 1202, during implantation of support structure 1000 the guidewire 1202 may be inserted first as is generally depicted at FIG. 12 at 12A and at FIG. 13 at 13A. Nipple 1006 may be threaded onto the proximal end of guidewire 1302 and the support structure 1000 may be advanced into the blood pump assembly 300 over guidewire 1202 as depicted in 12B-C and 13B-C. Once support structure 1000 is fully disposed in assembly 300, guidewire 1302 may be removed, as is generally depicted in 12D and 13D. Although not depicted, connector 602, 702 may then be installed as described above with respect to FIGs. 8-9.

In some embodiments, a sleeve (not depicted) may be utilized to advance support structure 1000 into the blood pump assembly 300. The process of using a sleeve is as described with respect to FIG. 3-4 and may occur either before or after the guidewire 1202 is inserted into the blood pump assembly 300. A lubricant may be used in connection with the sleeve on the inside, outside, or both inside and outside surfaces. Once support structure 1000 is in place, the sleeve may then be removed before or after extraction of the guidewire 1202.

To extract support structure 1000 using guidewire 1202, the inverse process may be performed. Optionally, if applicable, connector 602, 702 may be uninstalled. This may include removing cap 608 and/or releasing the tail region 1006 from clamp or lock, as is generally described with reference to FIG. 4. Guidewire 1202 may then be inserted in 14A with the distal end of guidewire 1202 threading nipple 1006. An external extraction force may be applied at the proximal end of tail region 1004 and the support structure 1000 may be explanted in 14B-C prior to removal of guidewire 1202 in 14D. Although not depicted, explantation may utilize a sleeve (not depicted) which may be first inserted into assembly 300 over support structure 100 (e.g., using a lubricant) before or after the guidewire is inserted. After support structure 1000 is explanted, the sleeve may be removed before or after the guidewire 1202 is extracted.

A such, guidewire 1202 may act to facilitate implantation and explantation of structure 200, 1000 into blood pump assembly 300. During implantation and explantation, the guidewire 1202 may support structure 200, 1000.

When disposed in assembly 300, support structure 200, 1000 may oppose forces acting on it in the descending aorta 110 when in operation (e.g., during counterpulsation) that might otherwise cause balloon 302 to roll or fold upon itself along the longitudinal axis of balloon, the transverse axis of balloon 302, or some angle with respect to either or both axes.

FIG. 15 illustrates a side view of an exemplary blood pump support apparatus in the form of a support structure 1500 configured to be removably disposed in a blood pump assembly, the support structure 1500 having head region 1501 and a tail region 1506, the head region 1501 having a flexible tip portion 1502 and a non-flexible body portion 1504. The flexible tip portion 1502 may be the distal portion of the head region 1501 and the non-flexible body portion 1504 may be proximal portion of the head region 1501. Flexible tip region 1502 may be flexible and atraumatic. Flexible tip region 1502 is depicted in a first position 1502A and in a second alternate position 1502B. Other positions are contemplated. A tail region 1506 is coupled to the proximal end of head region 1501. Flexible tip portion 1502 (or at least its core) may be tapered relative to the non-flexible body portion 1504 to provide the flexibility described herein. For example, the flexible tip portion 1504 may taper approximately for 1.5 cm to 8 cm from its distal end. The support structure 1500 may be composed of Nitinol or stainless steel. In some embodiments, the Nitinol or stainless steel is the core of the support structure 1500 and a wire, jacket, and/or coating may be applied as one or more layers to the core. For example, a flat or round wire may be coiled around the core or a polymer jacket with hydrophilic coating may be applied. PTFE coatings may also be applied (e.g., to minimize friction). In other embodiments other materials may be used. In one embodiment, only a core (e.g., a metal or metal alloy core) of flexible tip portion 1502 is tapered relative to the core of the non-flexible body portion 1504 and the wire, jacket, and/or other coatings applied over the core may be such the head region 1501 or head region 1501 and tail region 1506 have the same or substantially the same exterior shape and size. For example, if support structure 1500 has a circular cross section, the diameter of the cross section across support structure 1500 is substantially the same, i.e., it does not vary by more than 10%. The distal tip of flexible tip region 1502 may be angled, straight, j-shaped, or baby J-shaped.

In one embodiment, all or a portion of the support structure 1500 is radiopaque. In one embodiment, radiopaque material 1508 (e.g., additional radiopaque material) is disposed on support structure 1500 (e.g., at the distal end of the tail region 1506). Radiopaque material 1508 may alternatively be disposed elsewhere along the head region 1501 and the tail region 1506. Radiopaque material 1508 may be used to align the support structure 1500 to the balloon or assembly into which it support structure 1500 is implanted (e.g., to ensure adequate insertion/to prevent over insertion of the support structure 1500). For example, corresponding radiopaque material may be disposed on balloon or the blood pump assembly and it may be desirable to align radiopaque material on support structure with corresponding radiopaque material on the balloon/assembly.

As was described in connection with FIG. 11, non-radiopaque material 1112 may be added to a location on the support structure 1500, for example to a location on the tail region 1506. In one example, the non-radiopaque material is disposed at a proximal end of tail region 1506. The non-radiopaque material 1112 may serve as a visual marker, such that when the non-radiopaque material 1112 is aligned with a predetermined location of driveline 304 (or visible at or proximate a location external to the driveline 304) there is provided an indication that support structure 1500 or the head region 1501 is properly and fully inserted into balloon 302. For example, non-radiopaque material 1112 may be disposed on a location of the tail region 1506 that corresponds to a proximal end of driveline 304 when support structure 1500 is properly and fully inserted and deployed within balloon 302. In another example, the proximal end of driveline 304 may be trimmable at a location at or external to the axillary/subclavian artery 112 such that when the support structure 1500 or the head region 1501 is properly and fully inserted and deployed within balloon 302, the non-radiopaque material 1112 may be located at or proximate the trimmed location (e.g., the non-radiopaque material is aligned with the trimmer location). The non-radiopaque material 1112 may be disposed at any location on structure 1500 or tail region 1506.

FIG. 16 illustrates an implantation 16-B of the support structure 1500 of FIG. 15 into a blood pump assembly 1600 comprising a balloon 302 and a driveline 304 in accordance with an embodiment of the present disclosure. Blood pump assembly 1600 may be identical to blood pump assembly 300 but may include one or more radiopaque markers 1602 (e.g., disposed at the distal end of driveline 304 or elsewhere). A lubricant may be used and/or the balloon 302 may be partially inflated (e.g., at 20%) to facilitate implantation of support structure 1500 into the blood pump assembly 1800. As the head region 1501 of support structure 1500 is advanced through the driveline 304, the distal end of the flexible tip portion 1502 may make contact with the distal end of balloon 302 causing the flexible tip portion 1502 to curl upon itself. The flexible tip portion 1502 may be sufficiently flexible such that when such contact is made, flexible tip potion 1502 does not damage balloon 302 (e.g., an interior surface of balloon 302).

When the head region 1501 is disposed entirely in the balloon 302 (as may be determined when radiopaque markers 1608 and 1602 are aligned), the proximal end 1503 of the flexible tip portion 1502 may be curved (e.g., approximately 180 degrees) such that the flexible tip portion 1502 is curled back away from the distal end of the balloon 302 with the distal end 1505 of the flexible tip portion 1502 being disposed at a longitudinal position along the balloon 302 away from distal end of balloon 302. In this position the flexible tip portion 1502 may resemble a tail. The flexible tip potion 1502 may be flexible to insertion forces that cause it to curl as described above and in at least one embodiment sufficiently rigid to minimize or oppose forces acting on it in the descending aorta 110 when in operation (e.g., during counterpulsation) that might otherwise cause balloon 302 to roll or fold upon itself along the transverse axis of balloon 302, or some angle with respect to such transverse axis. The non-flexible body portion 1504 may resist or oppose, at least in part, forces acting on it in the descending aorta 110 when in operation (e.g., during counterpulsation) that might otherwise cause balloon 302 to roll or fold upon itself along the longitudinal axis of balloon 302, or some angle respect to such longitudinal axis.

FIG. 17 illustrates an exemplary explantation of the support structure 1500 from blood pump assembly 1600. The process is the reverse of the insertion process described in connection with FIG. 16.

FIG. 18 illustrates support structure 1500 disposed within assembly 1600 with a connector 602, 702 coupled to the proximal end of driveline 304 in accordance with one embodiment of the disclosure. Connector 602, 702 may be installed and removed as is described above with respect to support structures 200, 1000, and may operate to prevent operational forces from driving support structure 1500 out of assembly 1600 (e.g., connector 602, 702 may prevent structure 1500 from backing out of assembly 1600). A clamp associated with the connector 602 may clamp down on support structure 1500 (e.g., at a proximate end of tail region 1506) or an inside surface of removable cap 607 or fixed cap 706 may contain support structure 1500 inside assembly 300 as described above.

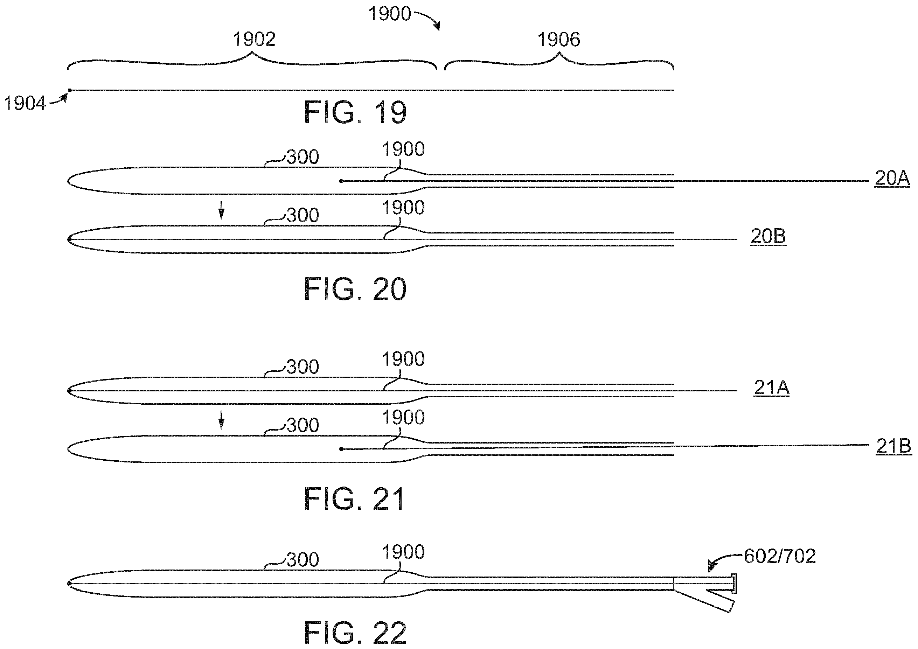

FIG. 19 illustrates a side view of an exemplary blood pump support apparatus in the form of a support structure 1900 configured to be disposed in a blood pump assembly, the support structure 1900 having head region 1902 with a blunted tip 1904 and a tail region 1906 in accordance with an embodiment of the present disclosure. In one embodiment, blunted tip 1902 is a ball-like surface. Support structure 1900 may be comprised of Nitinol. In other embodiments, support structure 1900 may be comprised of stainless steel. Support structure 1900 may include radiopaque material as described above in connection with FIG. 11 or FIG. 15 for purposes of alignment of the structure 1900 into assembly 300. Similarly, support structure 1900 may include non-radiopaque material as was described in connection with FIG. 11 for purposes of alignment of the structure 1900 into assembly 300.

FIG. 20 illustrates an implantation of the support structure 1900 into assembly 300 comprising balloon 302 and driveline 302 in accordance with an embodiment of the present disclosure. During implantation, the head region 1902 may be inserted into the balloon 203 with the blunted tip 1904 disposed at the distal end of balloon 302.

FIG. 21 illustrates an exemplary explantation of the support structure 1900 from blood pump assembly 300. Explantation may be the reverse process of implantation.

FIG. 22 illustrates an exemplary support structure 1900 disposed within a blood pump assembly 300 comprising balloon 302, a driveline 304. Connector 602, 702 may be installed and removed as is described above with respect to support structures 200, 1000, and may operate to prevent operational forces from driving support structure 1900 out of assembly 300 (e.g., connector 602, 702 may prevent structure 2200 from backing out of assembly 300). A clamp associated with the connector 602 may clamp down on support structure 1900 (e.g., at a proximate end of tail region 1906) or an inside surface of removable cap 607 or fixed cap 706 may contain support structure 1900 inside assembly 300 as described above.

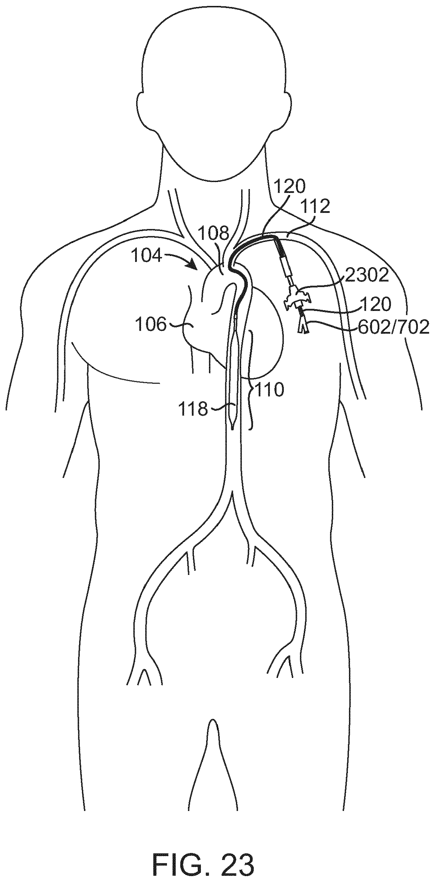

FIG. 23 illustrates the use of an exemplary arterial interface of stopper device 2502 to provide hemostasis at the arteriotomy site associated with a balloon implanted using an axillary implantation technique. Balloon 108 may be disposed in the descending aorta 110 using an axillary implantation technique such that the driveline 110 extends through the axillary/subclavian artery 112. Balloon 108 and driveline 110 may be part of a blood pump assembly of the types described herein. An arterial interface device or stopper device 2502 (as described above) may provide hemostasis at the arteriotomy where driveline 110 exists the axillary/subclavian artery 112. The arterial interface or stopper device 2502 may be deployed or advanced over the driveline 120 (e.g., that portion that is externalized from the vasculature) through a shaft in device 2502 that defines a lumen. Driveline 120 may be inserted into and extend through the shaft. Connector 602, 702 may be coupled to the proximal end of driveline 120.

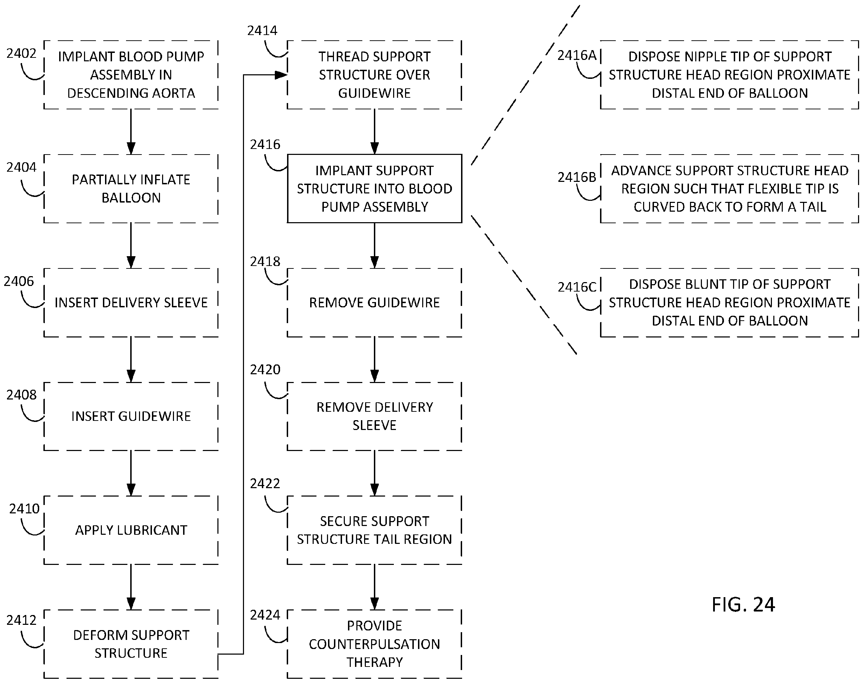

FIG. 24 illustrates an exemplary blood pump support method. Optional elements of the method are indicated in dashed boxes. The features of the method may be performed in the order presented in the figure. Alternatively, the features may be performed in a different order. For example at least blocks 2406-2414, to the extent they are implemented, may be performed in any order. Similarly, at least blocks 2418 and 2420, to the extent they are implemented, may be performed in any order.

The method may start with implanting a blood pump assembly (e.g., assembly 300) into a patient's descending aorta at block 2402 using, for example a femoral or axillary/subclavian technique. The method may proceed, at block 2404, where the balloon, e.g., balloon 302 of assembly 300 may be partially inflated. For example, balloon may be inflated to 50% of its volume. Next, a delivery sleeve may be inserted (e.g., into the blood pump assembly) at block 2406. The sleeve may be inserted into the driveline and/or into the balloon. A guidewire (e.g., guidewire 1202) may be inserted at block 2408. At block 2410, lubricant may be applied (e.g., to the sleeve and/or support structure (e.g., structures 200, 1000, 1500, and/or 1900). At block 2414, a support structure (e.g., structure 200, 1000) may be deformed (e.g., by placing it in a loaded state). The support structure (e.g., structure 200, 1000) may be threaded over the guidewire (e.g., guidewire 1202) at block 2414. In particular nipple tip 206, 1006 may be threaded over guidewire 1202.

In block 2416, the support structure (e.g., structure 200, 1000, 1500, 1900) may be implanted into the blood pump assembly (e.g., assembly 300). Block 2416 may include block 2416A, where the method includes disposing the nipple tip (e.g., nipple tip 206, 1006) proximate the distal end of balloon (e.g., balloon 302). The nipple tip may be touchingly adjacent or not touchingly adjacent to an inside distal end of balloon. By so disposing the nipple tip, the head region of structure (e.g., head region 202, 1002 of structure 200, 1000) may be disposed in balloon 302. Block 2416 may also include block 2416B where the structure is advanced into the balloon until a flexible tip is curved back to form a tail. For example, as depicted in FIG. 16, head region 1501 of structure 1500 may be advanced into balloon 302 such that a proximal end of flexible tip portion 1502 makes contact with an interior distal wall of the balloon and curves back onto itself. Block 2416 may also include block 2416C where a blunt tip of a support structure is disposed proximate the distal end of balloon. For example, as depicted in FIG. 20, structure 1900 may be advanced until the blunt tip 1904 is touchingly adjacent or not touchingly adjacent to an inside distal end of balloon 302. In each case, performance of any or all of blocks 2416A-C may permit the structure in operation to oppose forces that may otherwise cause balloon to roll or fold upon itself (or otherwise develop crevices) along the balloon's longitudinal axis, transverse axis, or an angle with respect to such axes.

In one embodiment, one or more radiopaque markers (e.g., markers 1102-1110) is disposed along the support structure (e.g., support structure 200, 1000, 1500, 1900). The one or more radiopaque markers may be disposed in one or more of the following locations: at the distal end of the head region, at a midpoint of the head region, at a proximal endo of the head region, at a distal end of the tail region, at a first midpoint of the tail region, and at a second midpoint of the tail region. Block 2416 may further include implanting the support structure into the blood pump assembly until at least one of the one or more radiopaque markers are aligned with one or more corresponding radiopaque markers on the blood pump assembly.

In another embodiment, a non-radiopaque material is disposed at a location on the support structure. Block 2416 may further include implanting the support structure until the non-radiopaque material is aligned with a predetermined location of the driveline. The non-radiopaque material may be a visual marker and the predetermined location of the driveline may be the proximal end of the driveline.

The method may continue in block 2418 where the guidewire is removed. For example guidewire 21202 may be extracted from assembly 300. A delivery sleeve may be similarly removed (e.g., from assembly 300) in block 2420. And in block 2422, the tail region of support structure may be secured. For example, in structures 200, 1000 the stems 212, 1012 of tail region 204, 1004 may be secured. In structure 1500, tail region 1506 may be secured. And in structure 1900, tail region 1906 may be secured. In each case, a connector may be used to secure the applicable tail region. For example, connector 602 or connector 702 may be installed on a proximal end of the driveline 304 of assembly 300 and the tail region may be secured by clamping or containing the tail region. By securing the tail region, the structure may be prevented from backing out of assembly due to operational forces acting on the balloon and the structure disposed therein. Finally, the method may include block 2424 where counterpulsation therapy may be provided using the blood pump assembly having a blood pump support apparatus implanted therein. The blood pump support apparatus may include a support structure (e.g., structure 200, 1000, 1500, 1900) implanted within a balloon of assembly (e.g., balloon 302) and may further include a connector coupled to a driveline of assembly (e.g., driveline 304) that may secure a tail region of the support structure.

### Conclusion

The above detailed description of embodiments of the technology are not intended to be exhaustive or to limit the technology to the precise forms disclosed above. Although specific embodiments of, and examples for, the technology are described above for illustrative purposes, various equivalent modifications are possible within the scope of the technology as those skilled in the relevant art will recognize. The various embodiments described herein may also be combined to provide further embodiments.

From the foregoing, it will be appreciated that specific embodiments of the technology have been described herein for purposes of illustration, but well-known support structures and functions have not been shown or described in detail to avoid unnecessarily obscuring the description of the embodiments of the technology.

Where the context permits, singular or plural terms may also include the plural or singular term, respectively. As used herein, the phrase "and/or" as in "A and/or B" refers to A alone, B alone, and A and B. Additionally, the term "comprising" is used throughout to mean including at least the recited feature(s) such that any greater number of the same feature and/or additional types of other features are not precluded. Further, any ranges identified herein are intended to be inclusive of the numbers that define the range, whether expressly stated or not. The same applies to approximate ranges. It will also be appreciated that specific embodiments have been described herein for purposes of illustration, but that various modifications may be made without deviating from the technology. Further, while advantages associated with some embodiments of the technology have been described in the context of those embodiments, other embodiments may also exhibit such advantages, and not all embodiments need necessarily exhibit such advantages to fall within the scope of the technology. Accordingly, the disclosure and associated technology may encompass other embodiments not expressly shown or described herein.

From the foregoing, it will be appreciated that specific embodiments of the invention have been described herein for purposes of illustration, but that various modifications may be made without deviating from the scope of the invention. For example, although only discussed in connection with support structure 1500, each of support structures 200, 1000, 1500, and 1900 may comprise a core material (e.g., Nitinol or stainless steel) and may further comprise a wire, jacket, and/or coating that is applied as one or more layers to the core. For example, a flat or round wire may be coiled around the core or a polymer jacket with hydrophilic coating may be applied. PTFE coatings may also be applied (e.g., to minimize friction). Accordingly, the invention is not limited except as by the appended claims.

By implanting or disposing a support structure into a blood pump assembly having a balloon and driveline, the support structure opposes forces that may otherwise cause balloon to roll or fold upon itself (or otherwise develop crevices) along the balloon's longitudinal axis, or an angle with respect to such axis. In at least some embodiments, the support structure further opposes forces that would otherwise cause the balloon to roll or fold upon itself (or otherwise develop crevices) along the balloon's transverse axis, or an angle with respect to such axis. This solves a problem associated with using thin-walled IABPs for mechanical circulatory support devices (e.g., counterpulsation). Preferred non-limiting embodiments of the present invention will now be described by way of reference to the clauses below.
1. A blood pump support apparatus for a blood pump assembly having an inflatable balloon coupled to a driveline, the driveline configured to shuttle a working fluid into and out of the inflatable balloon during operation such that when the blood pump assembly is implanted in the descending aorta of a patient, the blood pump assembly provides circulatory support to the patient, the blood pump support apparatus comprising:
   a support structure having a head region and a tail region, the support structure being configured to be removably disposable in the blood pump assembly such that the head region is disposed in the balloon; and
   the head region is configured to oppose forces that would otherwise cause the balloon to roll or fold upon itself along an angle with respect to the balloon's longitudinal axis, when the blood pump assembly is implanted in the descending aorta and the head region is disposed in the balloon.
2. The blood pump support apparatus of clause 1, wherein the support structure is removably disposable in the blood pump assembly via implantation through the driveline.
3. The blood pump support apparatus of clause 1, wherein when the head region is disposed in the balloon, the tail region is disposed in the driveline.
4. The blood pump support apparatus of clause 1, wherein, when the blood pump assembly is implanted in the descending aorta and the support structure is disposed in the blood pump assembly, the head region is configured to oppose forces that would otherwise cause the balloon to roll or fold upon itself along an angle with respect to the balloon's transverse axis.
5. The blood pump support apparatus of clause 1, wherein the support structure comprises a wire.
6. The blood pump support apparatus of clause 5, wherein the wire is one of Nitinol and stainless steel.
7. The blood pump support apparatus of clause 1, wherein the head region comprises one of Nitinol and stainless steel.
8. The blood pump support apparatus of clause 1, wherein the support structure is radiopaque.
9. The blood pump support apparatus of clause 1, wherein when the head region is disposed in the balloon, the tail region is disposed in the driveline and the proximal end of the tail region is external to the proximal end of the driveline.
10. The blood pump support apparatus of clause 9, further comprising a connector having a distal end that is removably coupleable to a proximal end of the driveline, the connector being configured to secure the tail region of the support structure.
11. The blood pump support apparatus of clause 10, wherein the connector comprises:
   an anti-backout port configured to receive a proximal end of the tail region of the support structure; and
   a clamp, wherein the clamp is configured to secure the proximal end of the tail region of the support structure.
12. The blood pump support apparatus of clause 10, wherein the connector comprises a cap and a proximal end of the tail region terminates proximate an inside surface of a cap, thereby containing the proximal end of the tail region.
13. The blood pump support apparatus of clause 12, wherein the proximal end of the tail region includes a flange.
14. The blood pump support apparatus of clause 10, wherein the cap is removably disposed on the proximal end of the anti-backout port.
15. The blood pump support apparatus of clause 10, wherein:
   the driveline has a lumen, and
   the connector include a pneumatic port that is in fluid communication with the driveline lumen.
16. The blood pump support apparatus of clause 15, wherein when the head region is disposed in the blood pump assembly, the tail region is configured to be disposed in the driveline lumen.
17. The blood pump support apparatus of clause 16, wherein the connector is a y-connector.
18. The blood pump support apparatus of clause 1, wherein:
   the head region is configured as a contiguous open-ended loop having two proximal ends and the tail region is configured as two stem ends, the distal end of each stem ends being contiguous with a respective proximal end of the head region,
   a width of the profile of the loop is greater than a width of the driveline,
   the head region exhibits elasticity, superelasticity or shape memory such that the head region can be reversibly deformed sufficient to implant the head region into the balloon through the driveline.
19. The blood pump support apparatus of clause 18, wherein the shape of the head region when disposed in the balloon prevents the head region from backing out of the balloon without the application of an external extraction force applied at the tail region.
20. The blood pump support apparatus of clause 18, wherein the distance between the two stem ends is less than the interior width of the driveline.
21. The blood pump support apparatus of clause 1, wherein the distal end of the head region comprises a nipple that is sized and shaped to be threaded by a guidewire.
22. The blood pump support apparatus of clause 21, wherein the profile of the head region tapers distally toward the distal end of the head region to form the nipple.
23. The blood pump support apparatus of clause 22, wherein the head region is crossed over itself at a base of the nipple.
24. The blood pump support apparatus of clause 18, wherein the proximate end of each stem end is a flange.
25. The blood pump support apparatus of clause 24, wherein the flanges are at an angle with respect to the transverse axis of the support structure.
26. The blood pump support apparatus of clause 18, wherein the stem ends are configured to be external to the proximal end of the driveline when the head region is disposed in the blood pump assembly.
27. The blood pump support apparatus of clause 1, wherein one or more radiopaque markers is disposed along the support structure..
28. The blood pump support apparatus of clause 1, wherein one or more non-radiopaque materials is disposed at a location on the support structure such that when the one or more non-radiopaque material are aligned with a predetermined location of the driveline, it is determined that head region is disposed in the balloon.
29. The blood pump support apparatus of clause 28, wherein the one or more non-radiopaque materials are visual markers and the predetermined location of the driveline is a proximal end of the driveline.
30. The blood pump support apparatus of clause 1, wherein the head region comprises a flexible tip portion and a non-flexible body portion, the flexible tip portion comprising the distal portion of the head region and the non-flexible body portion comprising the proximal portion of the head region.
31. The blood pump support apparatus of clause 30, wherein the head region is configured such that:
   the distal end of the non-flexible body portion is capable of being disposable proximate the distal end of the balloon,
   the proximal end of the flexible tip portion is configured to be curved, and
   the flexible tip portion is configured to be curled back away from the distal end of the balloon with the distal end of the flexible tip portion being disposed at a longitudinal position along the balloon.
32. The blood pump support apparatus of clause 31, wherein at least one radiopaque marker is disposed along one of the head region and the tail region that corresponds to a complementary radiopaque marker disposed at a location along one of the balloon and the driveline such that when the radiopaque markers are aligned it is determined that head region is disposed in the balloon.
33. The blood pump support apparatus of clause 1, wherein one or more non-radiopaque materials is disposed at a location on the support structure such that when the one or more non-radiopaque material are aligned with a predetermined location of the driveline, it is determined that head region is disposed in the balloon.
34. The blood pump support apparatus of clause 33, wherein the one or more non-radiopaque materials are visual markers and the predetermined location of the driveline is a proximal end of the driveline.
35. The blood pump support apparatus of clause 31, wherein the flexible tip portion is atraumatic and at least the core of the flexible tip portion is tapered relative to at least one of the non-flexible body portion and the tail region.
36. The blood pump support apparatus of clause 1, wherein the support structure has a blunt surface disposed at the distal end of the head region.
37. A blood pump support method for a blood pump assembly having an inflatable balloon coupled to a driveline, the driveline configured to shuttle a working fluid into and out of the inflatable balloon during operation such that when the blood pump assembly is implanted in the descending aorta of a patient, the blood pump assembly provides circulatory support to the patient, the blood pump support method comprising:
   implanting a support structure in the blood pump assembly such that a head region of the support structure is disposed in the balloon and a tail region of the support structure is disposed in the driveline,
   wherein when the blood pump assembly is implanted in the descending aorta and the head region is disposed in the balloon, the head region is configured to oppose forces that would otherwise cause the balloon to roll or fold upon itself along an angle with respect to at least one of the balloon's longitudinal and transverse axes,.
38. The blood pump support method of clause 37, the method further comprising implanting the blood pump assembly in the descending aorta.
39. The blood pump support method of clause 37, the method further comprising at least partially inflating the balloon prior to implanting the support structure.
40. The blood pump support method of clause 37, wherein implanting the support structure comprising implanting the support structure through the driveline.
41. The blood pump support method of clause 37, wherein the support structure comprises a wire.
42. The blood pump support apparatus of clause 41, wherein the wire is one of Nitinol and stainless steel.
43. The blood pump support method of clause 37, wherein implanting the support structure in the blood pump assembly comprises externalizing the proximal end of the tail region with respect to the proximal end of the driveline.
44. The blood pump support method of clause 43, further comprising:
   installing a connector on a proximal end of the driveline; and
   securing the tail region of the support structure using the connector.
45. The blood pump support method of clause 44, wherein:
   the connector comprises an anti-backout port, and
   securing the tail region comprises receiving the proximal end of the tail region of the support structure in the anti-backout port.
46. The blood pump support method of clause 44, wherein:
   the connector comprises a clamp, and
   securing the tail region comprises clamping the proximal end of the tail region using the clamp.
47. The blood pump support method of clause 45, wherein:
   the connector comprises a cap disposed on a proximal end of the anti-backout port, and
   securing the tail region comprises receiving the proximal end of the tail region of the support structure proximate an inside surface of the cap.
48. The blood pump support method of clause 45, wherein:
   the connector comprises a removable cap disposed on a proximal end of the anti-backout port.
49. The blood pump support method of clause 37, wherein:
   the head region is configured as a contiguous open-ended loop having two proximal ends and the tail region is configured as two stem ends, the distal end of each stem ends being contiguous with a respective proximal end of the head region,
   a width of the profile of the loop is greater than a width of the driveline,
   the head region exhibits elasticity, superelasticity or shape memory,
   implanting the support structure in the blood pump assembly comprises reversibly deforming the head region to advance the head region into the balloon through the driveline.
50. The blood pump support method of clause 49, wherein the shape of the head region when disposed in the balloon prevents the head region from backing out of the balloon without the application of an external extraction force applied at the tail region.
51. The blood pump support method of clause 37, wherein:
   the distal end of the head region comprises a nipple that is sized and shaped to be threaded by a guidewire; and
   implanting the support structure in the blood pump assembly comprises:
      inserting the guidewire into the blood pump assembly;
      threading the nipple onto the guidewire; and
      inserting the support structure into the blood pump assembly over the guidewire.
52. The blood pump support method of clause 51, wherein the profile of the head region tapers distally toward the distal end of the head region to form the nipple.
53. The blood pump support method of clause 52, wherein the head region is crossed over itself at a base of the nipple.
54. The blood pump support method of clause 37, wherein:
   one or more radiopaque markers are disposed along the support structure, and
   implanting the support structure in the blood pump assembly comprises advancing the support structure until at least one of the one or more radiopaque markers are aligned with one or more corresponding radiopaque markers on the blood pump assembly.
55. The blood pump support method of clause 37, wherein:
   a non-radiopaque materials is disposed at a location on the support structure; and
   implanting the support structure in the blood pump assembly comprises advancing the support structure until the non-radiopaque materials is aligned with a predetermined location of the driveline.
56. The blood pump support apparatus of clause 55, wherein the non-radiopaque material is a visual marker and the predetermined location of the driveline is a proximal end of the driveline.
57. The blood pump support method of clause 37, wherein:
   the head region comprises a flexible tip portion and a non-flexible body portion, the flexible tip portion comprising the distal portion of the head region and the non-flexible body portion comprising the proximal portion of the head region; and
   implanting the support structure in the blood pump assembly comprises advancing the head region into the balloon until:
      the distal end of the non-flexible body portion is disposed proximate the distal end of the balloon and
      the proximal end of the flexible tip portion is curved such that the flexible tip portion comprises a tail that is curled back away from the distal end of the balloon with the distal end of the flexible tip portion being disposed at a longitudinal position along the balloon.
58. The blood pump support apparatus of clause 57, wherein:
   at least one radiopaque marker is disposed along one of the head region and the tail region; and
   implanting the support structure in the blood pump assembly comprises advancing the support structure until the at least one radiopaque marker is aligned with at least one complementary radiopaque marker disposed on the blood pump assembly.
59. The blood pump support apparatus of clause 57, wherein the flexible tip portion is atraumatic and at least the core of the flexible tip portion is tapered relative to at least one of the non-flexible body portion and the tail region.
60. The blood pump support apparatus of clause 37, wherein:
   the support structure has a blunt surface disposed at the distal end of the head region; and
   implanting the support structure in the blood pump assembly comprises advancing the support structure until the blunt surface is proximate the inside surface of the distal end of the balloon.

Other preferred, non-limiting embodiments of the present invention will now be described by way of reference to the clauses below.
1. A blood pump support apparatus for a blood pump assembly having an inflatable balloon coupled to a driveline, the driveline configured to shuttle a working fluid into and out of the inflatable balloon during operation such that when the blood pump assembly is implanted in the descending aorta of a patient, the blood pump assembly provides circulatory support to the patient, the blood pump support apparatus comprising:
   a support structure having a head region and a tail region, the support structure being configured to be removably disposable in the blood pump assembly such that the head region is disposed in the balloon; and
   the head region is configured to oppose forces that would otherwise cause the balloon to roll or fold upon itself along an angle with respect to the balloon's longitudinal axis, when the blood pump assembly is implanted in the descending aorta and the head region is disposed in the balloon.
2. The blood pump support apparatus of clause 1, wherein the support structure is removably disposable in the blood pump assembly via implantation through the driveline.
3. The blood pump support apparatus of clause 1 or 2, wherein when the head region is disposed in the balloon, the tail region is disposed in the driveline.
4. The blood pump support apparatus of clause 1, 2 or 3, wherein, when the blood pump assembly is implanted in the descending aorta and the support structure is disposed in the blood pump assembly, the head region is configured to oppose forces that would otherwise cause the balloon to roll or fold upon itself along an angle with respect to the balloon's transverse axis.
5. The blood pump support apparatus of any one of the preceding clauses, wherein the support structure comprises a wire.
6. The blood pump support apparatus of clause 5, wherein the wire is one of Nitinol and stainless steel.
7. The blood pump support apparatus one of the preceding clauses, wherein the head region comprises one of Nitinol and stainless steel.
8. The blood pump support apparatus of any one of the preceding clauses, wherein the support structure is radiopaque.
9. The blood pump support apparatus of any one of the preceding clauses, wherein when the head region is disposed in the balloon, the tail region is disposed in the driveline and the proximal end of the tail region is external to the proximal end of the driveline.
10. The blood pump support apparatus of any one of clauses 1 to 9, further comprising a connector having a distal end that is removably coupleable to a proximal end of the driveline, the connector being configured to secure the tail region of the support structure.
11. The blood pump support apparatus of any one of clauses 1 to 10, wherein the connector comprises:
   an anti-backout port configured to receive a proximal end of the tail region of the support structure; and
   a clamp, wherein the clamp is configured to secure the proximal end of the tail region of the support structure.
12. The blood pump support apparatus of any one of clauses 1 to 10, wherein the connector comprises a cap and a proximal end of the tail region terminates proximate an inside surface of a cap, thereby containing the proximal end of the tail region.
13. The blood pump support apparatus of any one of clauses 1 to 12, wherein the proximal end of the tail region includes a flange.
14. The blood pump support apparatus of any one of clauses 1 to 13, wherein the cap is removably disposed on the proximal end of the anti-backout port.
15. The blood pump support apparatus of any one of the preceding clauses wherein:
   the driveline has a lumen, and
   the connector include a pneumatic port that is in fluid communication with the driveline lumen.
16. The blood pump support apparatus of any one of clauses 1 to 15, wherein when the head region is disposed in the blood pump assembly, the tail region is configured to be disposed in the driveline lumen.
17. The blood pump support apparatus of any one of clauses 1 to 16 wherein the connector is a y-connector.
18. The blood pump support apparatus of any one of the preceding clauses, wherein:
   the head region is configured as a contiguous open-ended loop having two proximal ends and the tail region is configured as two stem ends, the distal end of each stem ends being contiguous with a respective proximal end of the head region,
   a width of the profile of the loop is greater than a width of the driveline,
   the head region exhibits elasticity, superelasticity or shape memory such that the head region can be reversibly deformed sufficient to implant the head region into the balloon through the driveline.
19. The blood pump support apparatus of any one of clauses 1 to 18, wherein the shape of the head region when disposed in the balloon prevents the head region from backing out of the balloon without the application of an external extraction force applied at the tail region.
20. The blood pump support apparatus of any one of clauses 1 to 18, wherein the distance between the two stem ends is less than the interior width of the driveline.
21. The blood pump support apparatus of any one of the preceding clauses, wherein the distal end of the head region comprises a nipple that is sized and shaped to be threaded by a guidewire.
22. The blood pump support apparatus of any one of clauses 1 to 21, or any one of the preceding clauses, wherein the profile of the head region tapers distally toward the distal end of the head region to form the nipple.
23. The blood pump support apparatus of any one of clauses 1 to 22, wherein the head region is crossed over itself at a base of the nipple.
24. The blood pump support apparatus of any one of the preceding clauses, wherein the proximate end of each stem end is a flange.
25. The blood pump support apparatus of any one of clauses 1 to 24, wherein the flanges are at an angle with respect to the transverse axis of the support structure.
26. The blood pump support apparatus of any one of the preceding clauses, wherein the stem ends are configured to be external to the proximal end of the driveline when the head region is disposed in the blood pump assembly.
27. The blood pump support apparatus of any one of the preceding clauses, wherein one or more radiopaque markers is disposed along the support structure.
28. The blood pump support apparatus of any one of the preceding clauses, wherein one or more non-radiopaque materials is disposed at a location on the support structure such that when the one or more non-radiopaque material are aligned with a predetermined location of the driveline, it is determined that head region is disposed in the balloon.
29. The blood pump support apparatus of any one of clauses 1 to 28, wherein the one or more non-radiopaque materials are visual markers and the predetermined location of the driveline is a proximal end of the driveline.
30. The blood pump support apparatus of any one of the preceding clauses, wherein the head region comprises a flexible tip portion and a non-flexible body portion, the flexible tip portion comprising the distal portion of the head region and the non-flexible body portion comprising the proximal portion of the head region.
31. The blood pump support apparatus of any one of clauses 1 to 30, wherein the head region is configured such that:
   the distal end of the non-flexible body portion is capable of being disposable proximate the distal end of the balloon,
   the proximal end of the flexible tip portion is configured to be curved, and
   the flexible tip portion is configured to be curled back away from the distal end of the balloon with the distal end of the flexible tip portion being disposed at a longitudinal position along the balloon.
32. The blood pump support apparatus of any one of clauses 1 to 31, wherein at least one radiopaque marker is disposed along one of the head region and the tail region that corresponds to a complementary radiopaque marker disposed at a location along one of the balloon and the driveline such that when the radiopaque markers are aligned it is determined that head region is disposed in the balloon.
33. The blood pump support apparatus of any one of the preceding clauses, wherein one or more non-radiopaque materials is disposed at a location on the support structure such that when the one or more non-radiopaque material are aligned with a predetermined location of the driveline, it is determined that head region is disposed in the balloon.
34. The blood pump support apparatus of any one of clauses 1 to 33, wherein the one or more non-radiopaque materials are visual markers and the predetermined location of the driveline is a proximal end of the driveline.
35. The blood pump support apparatus of any one of clauses 1 to 31, wherein the flexible tip portion is atraumatic and at least the core of the flexible tip portion is tapered relative to at least one of the non-flexible body portion and the tail region.
36. The blood pump support apparatus of any one of the preceding clauses, wherein the support structure has a blunt surface disposed at the distal end of the head region.
37. A blood pump support method for a blood pump assembly having an inflatable balloon coupled to a driveline, the driveline configured to shuttle a working fluid into and out of the inflatable balloon during operation such that when the blood pump assembly is implanted in the descending aorta of a patient, the blood pump assembly provides circulatory support to the patient, the blood pump support method comprising:
   implanting a support structure in the blood pump assembly such that a head region of the support structure is disposed in the balloon and a tail region of the support structure is disposed in the driveline,
   wherein when the blood pump assembly is implanted in the descending aorta and the head region is disposed in the balloon, the head region is configured to oppose forces that would otherwise cause the balloon to roll or fold upon itself along an angle with respect to at least one of the balloon's longitudinal and transverse axes,.
38. The blood pump support method of clause 37, the method further comprising implanting the blood pump assembly in the descending aorta.
39. The blood pump support method of clause 37 or 38, the method further comprising at least partially inflating the balloon prior to implanting the support structure.
40. The blood pump support method of clause 37, 38 or 39, wherein implanting the support structure comprising implanting the support structure through the driveline.
41. The blood pump support method of any one of clauses 37 to 40, wherein the support structure comprises a wire.
42. The blood pump support apparatus of clause 41, wherein the wire is one of Nitinol and stainless steel.
43. The blood pump support method of any one of clauses 37 to 42, wherein implanting the support structure in the blood pump assembly comprises externalizing the proximal end of the tail region with respect to the proximal end of the driveline.
44. The blood pump support method of clause 43, further comprising:
   installing a connector on a proximal end of the driveline; and
   securing the tail region of the support structure using the connector.
45. The blood pump support method of clause 44, wherein:
   the connector comprises an anti-backout port, and
   securing the tail region comprises receiving the proximal end of the tail region of the support structure in the anti-backout port.
46. The blood pump support method of clause 44, wherein:
   the connector comprises a clamp, and
   securing the tail region comprises clamping the proximal end of the tail region using the clamp.
47. The blood pump support method of clause 45, wherein:
   the connector comprises a cap disposed on a proximal end of the anti-backout port, and
   securing the tail region comprises receiving the proximal end of the tail region of the support structure proximate an inside surface of the cap.
48. The blood pump support method of clause 45, wherein:
   the connector comprises a removable cap disposed on a proximal end of the anti-backout port.
49. The blood pump support method of any one of clauses 37 to 48, wherein:
   the head region is configured as a contiguous open-ended loop having two proximal ends and the tail region is configured as two stem ends, the distal end of each stem ends being contiguous with a respective proximal end of the head region,
   a width of the profile of the loop is greater than a width of the driveline,
   the head region exhibits elasticity, superelasticity or shape memory,
   implanting the support structure in the blood pump assembly comprises reversibly deforming the head region to advance the head region into the balloon through the driveline.
50. The blood pump support method of any one of clauses 37 to 49, wherein the shape of the head region when disposed in the balloon prevents the head region from backing out of the balloon without the application of an external extraction force applied at the tail region.
51. The blood pump support method of any one of clauses 37 to 50, wherein:
   the distal end of the head region comprises a nipple that is sized and shaped to be threaded by a guidewire; and
   implanting the support structure in the blood pump assembly comprises:
      inserting the guidewire into the blood pump assembly;
      threading the nipple onto the guidewire; and
      inserting the support structure into the blood pump assembly over the guidewire.
52. The blood pump support method of any one of clauses 37 to 51, wherein the profile of the head region tapers distally toward the distal end of the head region to form the nipple.
53. The blood pump support method of any one of clauses 37 to 52, wherein the head region is crossed over itself at a base of the nipple.
54. The blood pump support method of any one of clauses 37 to 53, wherein:
   one or more radiopaque markers are disposed along the support structure, and
   implanting the support structure in the blood pump assembly comprises advancing the support structure until at least one of the one or more radiopaque markers are aligned with one or more corresponding radiopaque markers on the blood pump assembly.
55. The blood pump support method of any one of clauses 37 to 54, wherein:
   a non-radiopaque materials is disposed at a location on the support structure; and
   implanting the support structure in the blood pump assembly comprises advancing the support structure until the non-radiopaque materials is aligned with a predetermined location of the driveline.
56. The blood pump support apparatus of any one of clauses 37 to 55, wherein the non-radiopaque material is a visual marker and the predetermined location of the driveline is a proximal end of the driveline.
57. The blood pump support method of any one of clauses 37 to 56, wherein:
   the head region comprises a flexible tip portion and a non-flexible body portion, the flexible tip portion comprising the distal portion of the head region and the non-flexible body portion comprising the proximal portion of the head region; and
   implanting the support structure in the blood pump assembly comprises advancing the head region into the balloon until:
      the distal end of the non-flexible body portion is disposed proximate the distal end of the balloon and
      the proximal end of the flexible tip portion is curved such that the flexible tip portion comprises a tail that is curled back away from the distal end of the balloon with the distal end of the flexible tip portion being disposed at a longitudinal position along the balloon.
58. The blood pump support apparatus of any one of clauses 37 to 57, wherein:
   at least one radiopaque marker is disposed along one of the head region and the tail region; and
   implanting the support structure in the blood pump assembly comprises advancing the support structure until the at least one radiopaque marker is aligned with at least one complementary radiopaque marker disposed on the blood pump assembly.
59. The blood pump support apparatus of any one of clauses 37 to 58, wherein the flexible tip portion is atraumatic and at least the core of the flexible tip portion is tapered relative to at least one of the non-flexible body portion and the tail region.
60. The blood pump support apparatus of any one of clauses 37 to 59, wherein:
   the support structure has a blunt surface disposed at the distal end of the head region; and
   implanting the support structure in the blood pump assembly comprises advancing the support structure until the blunt surface is proximate the inside surface of the distal end of the balloon.

## Claims

1. A blood pump support apparatus for a blood pump assembly having an inflatable balloon coupled to a driveline, the driveline configured to shuttle a working fluid into and out of the inflatable balloon during operation such that when the blood pump assembly is implanted in the descending aorta of a patient, the blood pump assembly provides circulatory support to the patient, the blood pump support apparatus comprising:
a support structure having a head region and a tail region, the support structure being configured to be removably disposable in the blood pump assembly such that the head region is disposed in the balloon; and
the head region is configured to oppose forces that would otherwise cause the balloon to roll or fold upon itself along an angle with respect to the balloon's longitudinal axis, when the blood pump assembly is implanted in the descending aorta and the head region is disposed in the balloon.

2. The blood pump support apparatus of claim 1, wherein the support structure is removably disposable in the blood pump assembly via implantation through the driveline.

3. The blood pump support apparatus of claim 1 or 2, wherein when the head region is disposed in the balloon, the tail region is disposed in the driveline.

4. The blood pump support apparatus of claim 1, 2 or 3, wherein, when the blood pump assembly is implanted in the descending aorta and the support structure is disposed in the blood pump assembly, the head region is configured to oppose forces that would otherwise cause the balloon to roll or fold upon itself along an angle with respect to the balloon's transverse axis.

5. The blood pump support apparatus of any one of the preceding claims,
wherein the support structure comprises a wire, and one or more of the support structure and the head region is radiopaque and comprises one of Nitinol and stainless steel.

6. The blood pump support apparatus of any one of the preceding claims,
wherein when the head region is disposed in the balloon, the tail region is disposed in the driveline and the proximal end of the tail region is external to the proximal end of the driveline.

7. The blood pump support apparatus of any one of claims 1 to 6, further comprising a connector having a distal end that is removably coupleable to a proximal end of the driveline, the connector being configured to secure the tail region of the support structure.

8. The blood pump support apparatus of any one of claims 1 to 7, wherein the connector comprises:
an anti-backout port configured to receive a proximal end of the tail region of the support structure; and
a clamp, wherein the clamp is configured to secure the proximal end of the tail region of the support structure.

9. The blood pump support apparatus of any one of claims 1 to 8, wherein the connector comprises a cap and a proximal end of the tail region terminates proximate an inside surface of a cap, thereby containing the proximal end of the tail region.

10. The blood pump support apparatus of any one of claims 1 to 9, wherein the proximal end of the tail region includes a flange.

11. The blood pump support apparatus of any one of claims 1 to 10, wherein the cap is removably disposed on the proximal end of the anti-backout port.

12. The blood pump support apparatus of any one of the preceding claims wherein:
the driveline has a lumen, and
the connector include a pneumatic port that is in fluid communication with the driveline lumen.

13. The blood pump support apparatus of any one of claims 1 to 12, wherein when the head region is disposed in the blood pump assembly, the tail region is configured to be disposed in the driveline lumen.

14. The blood pump support apparatus of any one of claims 1 to 13 wherein the connector is a y-connector.

15. The blood pump support apparatus of any one of the preceding claims, wherein:
the head region is configured as a contiguous open-ended loop having two proximal ends and the tail region is configured as two stem ends, the distal end of each stem ends being contiguous with a respective proximal end of the head region,
a width of the profile of the loop is greater than a width of the driveline,
the head region exhibits elasticity, superelasticity or shape memory such that the head region can be reversibly deformed sufficient to implant the head region into the balloon through the driveline.
